# EUROPEAN PATENT APPLICATION

(11) **EP 4 052 723 A1**
(43) Date of publication of application: **07.09.2022**
(21) Application number: 21382174.7
(22) Date of filing: 02.03.2021
(51) Int. Cl.: A61K 38/55, A61K 38/57

(54) **ALPHA-1 ANTITRYPSIN DOSING REGIMEN**

(71) Applicant: Grifols Worldwide Operations Limited, Dublin 22 (IE)
(72) Inventor: HORRILLO SAURA, RAQUEL, 08150 PARETS DEL VALLES (ES); COSTA RIEROLA, MONTSERRAT, 08150 PARETS DEL VALLES (ES); XIFRO COLLSAMATA, FRANCESC XAVIER, 17003 GIRONA (ES); CAMPRUBI GIMENEZ, SANDRA, 08174 SANT CUGAT DEL VALLES (ES)
(74) Representative: Durán-Corretjer, S.L.P.

(57) **Abstract**

The present invention is related to the use of alpha-1-antitrypsin as a therapeutic agent in patients that have suffered neural cell injury arising from traumatic or non-traumatic causes such as a ischemic stroke, cerebral hypoxia or spinal cord injuries. Alpha-1-antitrypsin administered in accordance with the treatment regimen of the present invention may also find utility in the treatment of neuropathic pain and the promotion of spinal cord revascularisation following injury thereto. Compositions comprising alpha-1 antitrypsin may be administered parenterally or by inhalation to the patients as part of an innovative dosing regimen.

## Description

### Field of the Invention

The present invention relates to the use of Alpha-1 Antitrypsin as a neuroprotective agent in patients that have suffered neural cell injury arising from traumatic or non-traumatic causes.

### Background

Injuries to the brain, spinal cord, and peripheral nervous system can manifest in immediate or chronic neurodegenerative effects that overwhelmingly alter the quality of the affected individual's life.

Spinal cord injury and peripheral nerve injury are predominantly caused by trauma. Brain injuries can be classified as hereditary, congenital, induced by birth, or acquired since birth/acquired brain injury (ABI). There are two types of ABI: traumatic and non-traumatic, with both resulting in changes to the brain's neuronal activity that affect the physical integrity, metabolic activity, or functional ability of nerve cells in the brain.

Traumatic brain injuries (TBI) are characterised by an alteration in brain function, or other evidence of brain pathology, caused by an external force. Traumatic impact injuries can be defined as non-penetrating or penetrating, and include falls, assaults, motor vehicle accidents and sports injuries. Non-Traumatic Brain Injuries (NTBI) cause damage to the brain through non-impact processes such as a lack of oxygen, exposure to toxins, pressure from a tumour, *etc.* Examples of NTBI include stroke, aneurysms and lack of oxygen supply to the brain caused by heart attacks.

Stroke is one of the most prevalent categories of NTBI and occurs when the blood supply to part of the brain is interrupted or reduced, preventing brain tissue from getting oxygen and nutrients. Neurodegenerative processes commence almost immediately, and brain cells begin to die in minutes. An ischemic stroke occurs when a blood vessel (artery) supplying blood to an area of the brain becomes blocked by a blood clot. A haemorrhagic stroke happens when an artery in the brain leaks or ruptures. Of the two, ischemic stroke is the most common form of stroke.

The pervasiveness of stroke in modern society produces an immense burden on health care infrastructure and spend. At present, recombinant tissue plasminogen activator (rtPA) is the only FDA-approved therapeutic agent for ischemic stroke. The major functions of rtPA are dissolving blood clots and promoting reperfusion. An alternative method for re-establishing a blocked blood flow is by a surgical intervention.

rtPA treatment has a narrow therapeutic time-window and as such its widespread applicability is limited. Moreover, although restoring perfusion via rtPA to the ischemic tissue is important, cascades of necrosis, apoptosis, and inflammation commence within minutes of severe oxygen deprivation. Increasingly, it is posited that genetically programmed neural cell death during post-ischemic tissue inflammation (which can last days to weeks) contributes significantly to the ultimate pathology because of delays in patient treatment/evaluation. As such, permanent neural and neuronal damage can arise before a patient even presents for evaluation.

There are many reports of molecules in the prior art that exert a neuroprotective effect so as to limit the damage caused by disorders such as strokes, and traumatic injuries to the brain and spinal cord. One such example is U.S. Patent Publication No. 2016008437 in the name of Grifols Worldwide Operations Ltd which discloses apo-transferrin as exerting a neuroprotective effect by modulating the activity of Hypoxia Inducible Factors (HIF) in a rat model of stroke. The inventors observed a neuroprotective effect for apo-transferrin manifesting in a decrease in the volume of the infarcted area in the rats treated with apo-transferrin when compared with control rats.

It is immediately apparent that the severe and debilitating effects of neurodegeneration associated with spinal cord injury, brain injury, and peripheral nerve injury warrant considerable focus and attention. As such, alternative therapies focussing on this need are highly desirable.

### Description of the Invention

The words "comprises/comprising" and the words "having/including" when used herein with reference to the present invention are used to specify the presence of stated features, integers, steps or components but do not preclude the presence or addition of one or more other features, integers, steps, components or groups thereof.

It should be appreciated by those skilled in the art that the specific embodiments disclosed herein should not be read in isolation, and that the present specification intends for the disclosed embodiments to be read in combination with one another as opposed to individually. As such, each embodiment may serve as a basis for modifying or limiting other embodiments disclosed herein.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "10 to 100" should be interpreted to include not only the explicitly recited values of 10 to 100, but also include individual value and sub-ranges within the indicated range. Thus, included in this numerical range are individual values such as 10, 11, 12, 13... 97, 98, 99, 100 and sub-ranges such as from 10 to 40, from 25 to 40 and 50 to 60, *etc.* This same principle applies to ranges reciting only one numerical value, such as "at least 10". Furthermore, such an interpretation should apply regardless of the breadth of the range or the characteristics being described.

### Treatment of the Invention

In a first aspect, the present invention provides for a pharmaceutical composition comprising alpha-1 antitrypsin for the treatment of a neural cell injury arising from at least one of a traumatic brain injury, a non-traumatic brain injury, a spinal cord injury, or a peripheral nerve injury
wherein the alpha-1 antitrypsin is administered at a dose of about 80 mg/kg/day to about 240 mg/kg/day as part of a multiple-dosage regimen.

As used herein, "treatment" refers to any reduction, lessening, or elimination of the symptoms caused by the neural cell injury. "Treatment" shall also be construed to encompass any delay in the deterioration in the patient's symptoms or well-being, versus an untreated patient. Such treatment may be effected prophylactically, prior to the onset of the disorder, or therapeutically, following the onset of the disorder.

In a further aspect, the present invention provides for a pharmaceutical composition comprising alpha-1 antitrypsin for promoting spinal revascularisation in patient suffering from a spinal cord injury,
wherein the alpha-1 antitrypsin is administered at a dose of about 80 mg/kg/day to about 240 mg/kg/day as part of a multiple-dosage regimen.

As used herein, "promoting spinal revascularisation" shall be construed as meaning directly or indirectly having a positive effect on the restoration of blood flow to the spinal cord and its associated nerves following injury thereto.

In yet a further aspect, the present invention provides for a pharmaceutical composition comprising alpha-1 antitrypsin for treating neuropathic pain in patient suffering from a spinal cord injury,
wherein the alpha-1 antitrypsin is administered at a dose of about 80 mg/kg/day to about 240 mg/kg/day as part of a multiple-dosage regimen.

Again, "treating" shall be construed as any reduction, lessening, or elimination of the symptoms of neuropathic pain. In one embodiment, the neuropathic pain is central neuropathic pain. In a further embodiment, the neuropathic pain is peripheral neuropathic pain.

Alpha-1 antitrypsin is a plasma-based serine protease inhibitor (serpin) having a principle role in the protection of lung tissues from degradative enzymes such as neutrophil elastase, which breaks down the structural protein elastin. Alpha-1 antitrypsin deficiency is a hereditary disorder in which a lack of functional alpha-1 antitrypsin leads to a chronic degradation of lung tissue manifesting in respiratory complications such as chronic obstructive pulmonary disease (COPD). Alpha-1 antitrypsin has many other monikers such as alpha-1 proteinase inhibitor, A1PI, AAT, A1AT, and α1-antitrypsin.

The present invention includes all wild type mammalian variants of alpha-1 antitrypsin within its scope. Particularly preferred is human alpha-1 antitrypsin (UniProtKB Seq. No. P01009) having the amino acid sequence set forth in SEQ ID NO: 1.

Alpha-1 antitrypsin utilised in the present invention may be plasma-derived or recombinant. Plasma-derived alpha-1 antitrypsin is purified from the blood plasma of blood donors by processes well known to those of skill in the art. Suitable commercial preparations of human plasma-derived alpha-1 antitrypsin illustrative of materials useful in the present invention include, without limitation, preparations sold under the brand names PROLASTIN, PROLASTIN C, ZEMAIRA, GLASSIA and ARALAST.

The present specification includes within its scope recombinant derivatives of alpha-1 antitrypsin that differ from the wild type amino acid sequence of the human protein outlined in SEQ ID NO: 1, by one or more substitutions, one or more deletions, or one or more insertions that may not materially alter the structure, or hydropathic nature of the recombinant protein relative to the wild type protein. Recombinant variants of alpha-1 antitrypsin within the scope of the present invention may additionally comprise at least one post translational modification, such as pegylation, glycosylation, polysialylation, or combinations thereof.

In one embodiment, the present invention contemplates recombinant variants of alpha-1 antitrypsin having one or more conservative substitutions relative to the wild type protein in SEQ ID NO: 1. A "conservative substitution" is one in which an amino acid is substituted for another amino acid that has similar properties, such that one skilled in the art of peptide chemistry would expect the secondary structure and hydropathic nature of the polypeptide to be substantially unchanged. Generally, change within the following groups of amino acids represent conservative changes: (1) ala, pro, gly, glu, asp, gin, asn, ser, thr; (2) cys, ser, tyr, thr; (3) val, ile, leu, met, ala, phe; (4) lys, arg, his; and (5) phe, tyr, trp, his. For example, the recombinant alpha-1 antitrypsin within the scope of the present invention may possess at least 90%, 95%, 96%, 97%, 98% or 99% homology with the wild type human alpha-1 antitrypsin protein outlined in SEQ ID NO: 1.

The skilled person will appreciate that recombinant proteins can be obtained utilising standard techniques well known in the art of protein expression, production and purification. Nucleic acid sequences of a recombinant protein of interest can be inserted in any expression vector suitable for expression in the elected host cell, e.g. mammalian cells, insect cells, plant cells, yeast, and bacteria.

As used herein, the term "expression vector" refers to an entity capable of introducing a protein expression construct into a host cell. Some expression vectors also replicate inside host cells, which increases protein expression by the protein expression construct. One type of vector is a "plasmid," which refers to a circular double stranded DNA loop into which additional DNA segments may be ligated. Other vectors include cosmids, bacterial artificial chromosomes (BAC) and yeast artificial chromosomes (YAC), fosmids, phage and phagemids. Another type of vector is a viral vector, wherein additional DNA segments may be ligated into the viral genome. Certain vectors are capable of autonomous replication in a host cell into which they are introduced (e.g., vectors having an origin of replication which functions in the host cell). Other vectors can be integrated into the genome of a host cell upon introduction into the host cell and are thereby replicated along with the host genome. Moreover, certain preferred vectors are capable of directing the expression of genes to which they are operatively linked.

Suitable bacterial cells include *Escherichia coli, Bacillus subtilis, Salmonella typhimurium, Pseudomonas* spp., *Streptomyces* spp., and *Staphylococcus* spp. Suitable yeast cells include *Saccharomyces* spp., *Pichia* spp., and *Kuyveromyces* spp. Suitable insect cells include those derived from *Bombyx mori, Mamestra brassicae, Spodoptera frugiperda, Trichoplusia ni* and *Drosophila melanogaster.* Such mammalian host cells include but are not limited to CHO, VERO, BHK, Hela, COS, MDCK, W138, BT483, Hs578T, HTB2, BT2O and T47D, NS0, CRL7O3O, HsS78Bst, human hepatocellular carcinoma cells (e.g. Hep G2), human adenovirus transformed 293 cells (e.g. HEK293), PER.C6, mouse L-929 cells, HaK hamster cell lines, murine 3T3 cells derived from Swiss, Balb-c or NIH mice, and CV-1 cell line cells.

The present invention also contemplates the use of wild type and recombinant alpha-1 antitrypsin proteins that are conjugated or fused to any other protein, protein fragment, protein domain, peptide, small molecule or other chemical entity. For example, suitable fusion or conjugation partners include serum albumins (for example, bovine, rabbit or human), keyhole limpet hemocyanin, immunoglobulin molecules (including the Fc domain of immunoglobulins), thyroglobulin, ovalbumin, tetanus toxoid, a toxoid from other pathogenic bacteria, an attenuated toxin derivative, cytokines, chemokines, glucagon-like peptide-1, exendin-4, XTEN, or combinations thereof.

In one embodiment, the alpha-1 antitrypsin having utility in the present invention may be fused to an immunoglobulin Fc domain. For example, the immunoglobulin Fc domain may comprise at least a portion of a constant heavy immunoglobulin domain. The constant heavy immunoglobulin domain is preferably an Fc fragment comprising the CH2 and CH3 domain and, optionally, at least a part of the hinge region. The immunoglobulin Fc domain may be an IgG, IgM, IgD, IgA or IgE immunoglobulin Fc domain, or a modified immunoglobulin Fc domain derived therefrom. Preferably, the immunoglobulin Fc domain comprises at least a portion of a constant IgG immunoglobulin Fc domain. The IgG immunoglobulin Fc domain may be selected from IgG1, IgG2, IgG3 or IgG4 Fc domains, or modified Fc domains thereof.

### Neural Cell Injury

Surprisingly, the present inventors have discovered that alpha-1 antitrypsin displays a non-linear dose response in the treatment of a neural cell injury arising from at least one of a traumatic brain injury, a non-traumatic brain injury, a spinal cord injury, or a peripheral nerve injury.

By "neural cell", the present specification includes all cells of the nervous system, including without limitation glial cells and neuronal cells. In one embodiment, the neural cells referred to in the treatment of the present invention are neuronal cells.

As used herein, the term "neural cell injury" shall mean any damage to a neural cell that results in the loss of structure and/or function of the neural cell, and includes the death of the neural cell. The injury may be an isolated one-off event/occurrence causing immediate neural cell damage or death. Alternatively, the injury may be a continuous or chronic event that progressively leads to increasing levels of neural cell damage or death.

The neural cell injury treated by alpha-1 antitrypsin may be caused by the consequences of at least one of a traumatic brain injury, a non-traumatic brain injury, a spinal cord injury, or a peripheral nerve injury. In one embodiment, the neural cell injury treated by alpha-1 antitrypsin may be caused by the consequences of at least one of a non-traumatic brain injury, or a spinal cord injury.

As used herein, the term "traumatic brain injury" refers to an injury to the brain caused by a penetrating or non-penetrating trauma to the head. There are many possible causes, and non-limiting examples include road traffic accidents, assaults, sporting collisions, unprotected falls and the like.

As used herein, the term "non-traumatic brain injury" refers to an injury to the brain resulting from a non-traumatic cause. Suitable, non-limiting examples of non-traumatic causes include tumours, strokes, transient ischemic attacks, brain haemorrhages, haemorrhagic strokes, ischemic strokes, cerebral hypoxia, cerebral anoxia, neonatal hypoxia, consumption of chemical toxins/drugs, hydrocephalus, meningitis and encephalitis.

In one embodiment, the neural cell injury treated by alpha-1 antitrypsin may be caused by a non-traumatic brain injury. In one embodiment, the non-traumatic brain injury is caused by a stroke, such as an ischemic stroke or a haemorrhagic stroke. For example, the non-traumatic brain injury may be caused by an ischemic stroke. In one embodiment, the non-traumatic brain injury is caused by at least one of cerebral hypoxia, cerebral anoxia, or neonatal hypoxia.

In one embodiment, the neural cell injury treated by alpha-1 antitrypsin may be caused by a spinal cord injury. By "spinal cord injury" the present specification is to be construed as meaning damage to any part of the spinal cord or nerves at the end of the spinal canal that result in a loss of function, such as mobility and/or feeling. Spinal cord injuries can be traumatic or non-traumatic. Non-limiting causes of spinal cord injury include trauma (car accident, gunshot, falls, *etc*.), disease (polio, spina bifida, *etc*.), infection and tumours. In one embodiment, the spinal cord injury is caused by trauma.

In one embodiment, the neural cell injury treated by alpha-1 antitrypsin may be caused by an ischemic event. The ischemic event may be a condition selected from the group consisting of an transient ischemic attack, ischemic stroke, cerebral hypoxia, cerebral anoxia, neonatal hypoxia, and combinations thereof. For example, the ischemic event may be a condition selected from the group consisting of ischemic stroke, and neonatal hypoxia. In one embodiment, the ischemic event is ischemic stroke.

It should be appreciated by those skilled in the art that the specific embodiments disclosed withinthis section (Neural Cell Injury) should not be read in isolation, and that the present specification intends for these embodiments to be disclosed in combination with other embodiments as opposed to being disclosed individually. For example, where the context does not imply otherwise, each of the embodiments disclosed in this section (Neural Cell Injury) is to be read as being explicitly combined with each of the embodiments in section Treatment of the Invention, or any permutation of 2 or more of the embodiments disclosed therein.

### Dosing & Administration

The alpha-1 antitrypsin administered as part of the dosage regimen of the invention may be administered directly or indirectly to the site of neural cell injury by any conventional drug delivery means known by those skilled in the art.

For example, the alpha-1 antitrypsin could be administered locally or proximate to the neural cell injury by a conventional route selected from the group consisting of intracerebral, intracranial, intraspinal, and intrathecal. Alternatively, the alpha-1 antitrypsin may be administered locally during surgical intervention.

Conversely, the alpha-1 antitrypsin could be delivered indirectly to the site of the neural cell injury by an administration route selected from the group consisting of intravenous, subcutaneous, intramuscular, intradermal, intraperitoneal, intrapulmonary, intranasal, transdermal, transmucosal, oral, vaginal and rectal.

In one embodiment, the alpha-1 antitrypsin is administered to the patient parenterally, for example intravenously. In another embodiment, the alpha-1 antitrypsin is administered to the patient by an administration route selected from intrapulmonary or intranasal, for example using a nebuliser or inhaler device.

The alpha-1 antitrypsin may be administered to the patient as part of a multiple-dosage regimen, wherein the alpha-1 antitrypsin is administered to the patient using the same route of administration for each dose. In one embodiment, each dose of alpha-1 antitrypsin is administered to the patient parenterally. For example, each dose of alpha-1 antitrypsin may be administered to the patient intravenously.

The present invention also provides for a dosage regimen that incorporates combined modes of administration. For example, the initial dose may be administered parenterally, such as intravenously or subcutaneously, and subsequent doses may be delivered by an administration route selected from intrapulmonary or intranasal. In an alternative embodiment, the first 1 to 5 doses may be administered parenterally, such as intravenously or subcutaneously, and subsequent doses may be by an administration route selected from intrapulmonary or intranasal.

Alternatively, the initial dose may be delivered by an administration route selected from intrapulmonary or intranasal, and subsequent doses may be administered parenterally, such as intravenously or subcutaneously. In another embodiment, the first 1 to 5 doses may be delivered by an administration route selected from intrapulmonary or intranasal, and subsequent doses may be administered parenterally, such as intravenously or subcutaneously.

In one embodiment, the amount of alpha-1 antitrypsin administered per dose is an amount between about 80 to about 240 mg/kg/day of body weight. In another embodiment, the amount of alpha-1 antitrypsin administered per dose is an amount between about 80 to about 180 mg/kg/day of body weight. In yet another embodiment, the amount of alpha-1 antitrypsin administered per dose is an amount between about 80 to about 140 mg/kg/day of body weight. In still another embodiment, the amount of alpha-1 antitrypsin administered per dose is an amount between about 80 to about 120 mg/kg/day of body weight. In another embodiment, the amount of alpha-1 antitrypsin administered per dose is an amount between about 90 to about 110 mg/kg/day of body weight. In one embodiment, the amount of alpha-1 antitrypsin administered per dose is about 100 mg/kg/day of body weight.

In one embodiment, the amount of alpha-1 antitrypsin administered to the patient may be the same for each dose during the course of the multiple-dosing regimen. For example, an initial dose of about 80 mg/kg to about 240 mg/kg on day 1 of an administration period, followed by the same dose of about 80 mg/kg/day to about 240 mg/kg/day per dose during a multiple dosing period. Such as, an initial dose of about 80 mg/kg to about 120 mg/kg on day 1 of an administration period, followed by the same dose of about 80 mg/kg/day to about 120 mg/kg/day per dose during a multiple dosing period. In one embodiment, an initial dose of about 100 mg/kg is administered on day 1 of an administration period, followed by doses of about 100 mg/kg/day per dose during a multiple dosing period.

In another embodiment, the amount of alpha-1 antitrypsin administered to the patient may vary from dose to dose over the course of the multiple-dosing regimen. For example, an initial dose of about 80 mg/kg to about 240 mg/kg on day 1 of an administration period, followed by about 80 mg/kg/day to about 180 mg/kg/day per dose during a multiple dosing period. Such as, an initial dose of about 80 mg/kg to about 180 mg/kg on day 1 of an administration period, followed by about 80 mg/kg/day to about 140 mg/kg/day per dose during a multiple dosing period. In one embodiment, an initial dose of about 80 mg/kg to about 140 mg/kg on day 1 of an administration period, followed by about 80 mg/kg to about 120 mg/kg per dose during a multiple dosing period.

The multiple dosing period may comprise from about 5 to about 30 administrations up to a total cumulative dose. For example, the multiple dosing period may comprise from about 5 to about 25 administrations up to a total cumulative dose. In one embodiment, the multiple dosing period may comprise from about 5 to about 20 administrations up to a total cumulative dose. In another embodiment, the multiple dosing period may comprise from about 5 to about 15 administrations up to a total cumulative dose. In yet a further embodiment, the multiple dosing period may comprise from about 4 to about 20 administrations up to a total cumulative dose. In one embodiment, the multiple dosing period may comprise from about 2 to about 20 administrations up to a total cumulative dose.

The multiple dosing period may extend over a period of about 1 to about 30 weeks. For example, the multiple dosing period may extend over a period of about 1 to about 20 weeks. In one embodiment, the multiple dosing period may extend over a period of about 1 to about 10 weeks. In one embodiment, the multiple dosing period may extend over a period of less than about 5 weeks. In yet a further embodiment, the multiple dosing period may extend over a period of less than about 3 weeks.

The multiple portion doses may be administered at equally spaced intervals of from about 1 day to about 30 days. For example, the multiple portion doses may be administered at equally spaced intervals of from about 1 day to about 15 days. In one embodiment, the multiple portion doses may be administered at equally spaced intervals of from about 1 day to about 10 days. In a further embodiment, the multiple portion doses may be administered at equally spaced intervals of from about 1 day to about 7 days.

Alternatively, the multiple portion doses may be administered at unequal intervals of from about 1 day to about 30 days. For example, the multiple portion doses may be administered at unequally spaced intervals of from about 1 day to about 15 days. In one embodiment, the multiple portion doses may be administered at unequally spaced intervals of from about 1 day to about 10 days. In a further embodiment, the multiple portion doses may be administered at unequally spaced intervals of from about 1 day to about 7 days.

As used herein, the term "unequally spaced intervals" shall mean that at least one of the multiple portion doses is administered at a different interval to the other doses. For example, the term shall be construed to include a dosing regimen in which the first 5 doses are administered at an interval of 1 day, and the subsequent 5 doses are administered at an interval of 7 days.

It should be appreciated by those skilled in the art that the specific embodiments disclosed within this section (Dosing & Administration)should not be read in isolation, and that the present specification intends for these embodiments to be disclosed in combination with other embodiments as opposed to being disclosed individually. For example, where the context does not imply otherwise, each of the embodiments disclosed in is to be read as being explicitly combined with each of the embodiments in section Treatment of the Invention, or any permutation of 2 or more of the embodiments disclosed therein.

### Pharmaceutical Compositions of the Invention

The pharmaceutical compositions of the present invention may optionally further comprise at least one pharmaceutically acceptable carrier. The at least one pharmaceutically acceptable carrier may be chosen from adjuvants and vehicles. The at least one pharmaceutically acceptable carrier, includes any and all solvents, diluents, other liquid vehicles, dispersion aids, suspension aids, surface active agents, isotonic agents, thickening agents, emulsifying agents, preservatives, as suited to the particular dosage form desired.

Suitable carriers are described in Remington: The Science and Practice of Pharmacy, 21 st edition, 2005, ed. D.B. Troy, Lippincott Williams & Wilkins, Philadelphia*, and* Encyclopedia of Pharmaceutical Technology, eds. J. Swarbrick and J. C. Boylan, 1988-1999, Marcel Dekker, New York*,* the contents of which are incorporated herein by reference. Preferred examples of such carriers or diluents include, but are not limited to, water, saline, Ringer's solutions, glycols, dextrose solution, buffered solutions (such as phosphates, glycine, sorbic acid, and potassium sorbate) and 5% human serum albumin. Liposomes and non-aqueous vehicles such as glyceride mixtures of saturated vegetable fatty acids, and fixed oils (such as peanut oil, cottonseed oil, safflower oil, sesame oil, olive oil, com oil and soybean oil) may also be used depending on the route of administration.

The pharmaceutical composition of the invention is formulated to be compatible with its intended route of administration. For systemic use, the pharmaceutical composition of the invention can be formulated for administration by a conventional route selected from the group consisting of intravenous, subcutaneous, intramuscular, intradermal, intraperitoneal, intracerebral, intracranial, intrapulmonary, intranasal, intraspinal, intrathecal, transdermal, transmucosal, oral, vaginal, and rectal.

In one embodiment, parenteral administration is the chosen route of administration. The pharmaceutical composition may be enclosed in ampoules, disposable syringes, sealed bags, or multiple dose vials made of glass or plastic. In one embodiment, administration as an intravenous injection is the chosen route of administration. The formulations can be administered continuously by infusion or by bolus injection.

Pharmaceutical compositions suitable for injectable use include sterile aqueous solutions (where water soluble) or dispersions, and sterile powders for the extemporaneous preparation of sterile injectable solutions or dispersions. For intravenous administration, suitable carriers include physiological saline, bacteriostatic water, CREMOPHOR EL, or phosphate buffered saline (PBS). In some embodiments, the carrier may be a solvent or dispersion medium containing, for example water, ethanol, polyols (for example, glycerol, propylene glycol, and liquid polyethylene glycol, and the like), and suitable mixtures thereof. In all cases, the composition must be sterile and should be fluid to the extent that easy syringe-ability exists.

Prevention of the growth of microorganisms can be achieved by various antibacterial and antifungal agents, for example parabens, chlorobutanol, phenol, ascorbic acid, thimerosal, and the like. In many cases, it will be preferable to include isotonic agents, for example sugars (such as mannitol, sorbitol, *etc*.), polyalcohols, or sodium chloride in the composition. Prolonged absorption of the injectable compositions can be brought about by including in the composition an agent which delays absorption, for example aluminum monostearate or gelatin.

Sterile injectable solutions of the pharmaceutical composition of the present invention can be prepared by incorporating the active molecule in the required amount in an appropriate solvent with one or a combination of ingredients as discussed above followed by filtered sterilization. In the case of sterile powders for the preparation of sterile injectable solutions, methods of preparation include vacuum drying and freeze-drying that provide a powder of the active ingredient plus any additional desired ingredient from a previously sterile-filtered solution thereof.

In one embodiment, the alpha-1 antitrypsin may constitute at least 20% by weight of the total protein content of the injectable pharmaceutical composition of the present invention. For example, the alpha-1 antitrypsin may constitute greater than or equal to about 30%, 40%, 50%, 60%, 70%, 75%, 80%, 85%, 90%, 93%, 95%, 96%, 97%, 98%, or 99% by weight of the total protein content of the injectable pharmaceutical composition of the present invention.

The injectable pharmaceutical compositions of the present invention may be presented as a unit dosage unit form, *i.e.* as physically discrete units intended as unitary dosages for the subject to be treated.

For example, aqueous formulations of alpha-1 antitrypsin suitable for subcutaneous administration may contain as excipients hydrophobic amino acids. Such aqueous formulations may have a near neutral pH of about 7. The hydrophobic amino acid may be an aliphatic hydrophobic amino acid. For example, the amino acid may be selected from the group consisting of alanine, leucine, isoleucine, and combinations thereof. In one embodiment, the amino acid is alanine.

In one embodiment, the present invention provides for aerosolized administration of alpha-1 antitrypsin. The aerosol may be a suspension of fine solid particles or liquid droplets in any pharmaceutically acceptable propellant gas. In one embodiment, the alpha-1 antitrypsin may be formulated as a dry powder suitable for use or atomisation in an inhalation device. The dry powder composition may contain excipients selected from the group consisting of glycine, trehalose, and combinations thereof. In one embodiment, the dry powder compositions of the present invention have a D90 particle size distribution of 30 microns or less, *ie* 90% of particles are smaller than 30 mm. The particle size distribution of the dry powder compositions of the present invention may be measured by any means know by those of skill in the art, such as light scattering.

In one embodiment, aqueous formulations of alpha-1 antitrypsin suitable for inhalation may contain as excipients phosphate buffered saline at a neutral pH of about 7.

In one embodiment, the alpha-1 antitrypsin may constitute at least 20% by weight of the total protein content of the dry powder pharmaceutical composition of the present invention. For example, the alpha-1 antitrypsin may constitute greater than or equal to about 30%, 40%, 50%, 60%, 70%, 75%, 80%, 85%, 90%, 93%, 95%, 96%, 97%, 98%, or 99% by weight of the total protein content of the dry powder pharmaceutical composition of the present invention.

It should be appreciated by those skilled in the art that the specific embodiments disclosed within this section (Pharmaceutical composition of the Invention)should not be read in isolation, and that the present specification intends for these embodiments to be disclosed in combination with other embodiments as opposed to being disclosed individually. For example, where the context does not imply otherwise, each of the embodiments disclosed in this section (Pharmaceutical composition of the Invention) is to be read as being explicitly combined with each of the embodiments in sections Treatment of the Invention, Neural Cell Injury, and Dosing & Administration, or any permutation of 2 or more of the embodiments disclosed therein.

### Combination Therapy

The present invention also contemplates the use of supplementary active compounds and molecules in combination with alpha-1 antitrypsin. The supplementary active compounds and molecules can be co-formulated with alpha-1 antitrypsin as a unit dosage form, *i.e.* as a physically discrete unit intended as a unitary dosage for the subject to be treated.

Alternatively, the supplementary active compounds and molecules can be presented as a kit-of-parts, and:
- administered separately to alpha-1 antitrypsin, in a phased or sequential dosing pattern; or
- co-administered simultaneously from different dosage forms.

For example, the present invention contemplates administering other serum, or plasma-based proteins in combination with alpha-1 antitrypsin. Serum or plasma proteins within the scope of the present invention include those purified from a suitable plasma source, such as human plasma, and those prepared using recombinant manufacturing techniques. For example, the serum or plasma protein may be selected from the group consisting of albumin (*e.g.* ALBUTEIN), antithrombin (*e.g.* THROMBATE III), polyclonal immunoglobulins (IgG, IgA, and combinations thereof), polyspecific immunoglobulins (IgM), C1 esterase inhibitor (e.g. BERINERT), transthyretin, transferrin, lactoferrin, and combinations thereof.

Exemplary polyclonal immunoglobulins within the scope of the present invention include commercially available polyclonal IgG formulations such as FLEBOGAMMA DIF 5% & 10%, GAMUNEX- C 10%, BIVIGAM 10%, GAMMAGARD Liquid 10%, *etc.*

Exemplary polyspecific immunoglobulins (IgM) within the scope of the present invention include commercially available immunoglobulin formulations containing polyspecific IgM such as PENTAGLOBIN or TRIMODULIN.

In one embodiment, the serum or plasma protein may be selected from the group consisting of albumin, antithrombin, transferrin, lactoferrin, and combinations thereof. For example, the serum or plasma protein may be selected from the group consisting of antithrombin, transferrin, lactoferrin, and combinations thereof.

In a particular embodiment, a therapeutically effective amount of antithrombin is administered to the patient in addition to alpha-1 antitrypsin.

In a particular embodiment, a therapeutically effective amount of protein selected from transferrin, lactoferrin, and combinations thereof is administered to the patient in addition to the alpha-1 antitrypsin. In one embodiment, a therapeutically effective amount of transferrin is administered to the patient in addition to alpha-1 antitrypsin.

In one embodiment, with respect to the combination therapy of the present invention, the alpha-1 antitrypsin may constitute at least 20% by weight of the total protein content utilised in the combination therapy of the present invention. For example, the alpha-1 antitrypsin may constitute greater than or equal to about 30%, 40%, 50%, 60%, 70%, 75%, 80%, 85%, 90%, 93%, 95%, 96%, 97%, 98%, or 99% by weight of the total protein content utilised in the combination therapy of the present invention.

The skilled person will appreciate that the present invention also contemplates within its scope covalent conjugates of each of the above listed serum or plasma proteins to alpha-1 antitrypsin. Furthermore, it will be appreciated that the present invention also contemplates within its scope recombinant fusion proteins of each of the above listed serum or plasma proteins to alpha-1 antitrypsin, optionally linked by an appropriate linker sequence.

In one embodiment, a therapeutically effective amount of transferrin or lactoferrin is administered to the patient in combination with alpha-1 antitrypsin. By "transferrin" and "lactoferrin" the present specification is to be construed as meaning a therapeutically effective amounts of:
- wild type (mammalian, preferably human) transferrin or lactoferrin proteins respectively,
- respective functional mutants thereof, or
- respective functional fragments thereof.

The present invention includes within its scope all wild type mammalian transferrin proteins, however, human transferrin (UniProtKB Seq. No. Q06AH7) with the amino acid sequence set forth in SEQ ID NO: 2 is particularly preferred. Similarly, the present invention includes within its scope all wild type mammalian lactoferrin proteins, however, human lactoferrin (UniProtKB Seq. No. P02788) with the amino acid sequence set forth in SEQ ID NO: 3 is particularly preferred.

The wild type transferrin protein contains two homologous lobes (N- and C-lobes) with each lobe binding a single iron atom. As such, each wild type transferrin molecule can bind up to two iron atoms or ions per molecule. Similarly, each wild type lactoferrin molecule can bind two iron atoms per molecule in an analogous fashion.

In one embodiment, the iron saturation of the transferrin, functional mutant thereof, or functional fragment thereof is about 50% or less. Preferably, the iron saturation is about 40% or less. In one embodiment, the iron saturation is about 30% or less. For example, the iron saturation may be about 20% or less, such as about 10% or less. In some embodiments, the iron saturation is about 5% or less. In yet a further embodiment, the iron saturation may be less than about 1%. For the avoidance of any doubt, ranges presented herein as less than X% include 0 to X%, *i.e.* transferrin with absolutely no bound iron - 0% iron saturation.

In one embodiment, the iron saturation of the lactoferrin, functional mutant thereof, or functional fragment thereof is about 50% or less. Preferably, the iron saturation is about 40% or less. In one embodiment, the iron saturation is about 30% or less. For example, the iron saturation may be about 20% or less, such as about 10% or less. In some embodiments, the iron saturation is about 5% or less. In yet a further embodiment, the iron saturation may be less than about 1%.

As used herein, "apo-transferrin" shall mean transferrin having an iron saturation of less than 1%. Similarly, "holo-transferrin" shall mean transferrin having an iron saturation of 99% or greater. As used herein, "apo-lactoferrin" shall mean lactoferrin having an iron saturation of less than 1%. Similarly, "holo-lactoferrin" shall mean lactoferrin having an iron saturation of 99% or greater.

The skilled person will appreciate that transferrin and lactoferrin iron saturation levels can be readily determined without undue burden by quantifying the total iron levels in a sample having a known protein concentration. The total iron levels in sample can be measured by any one of a number of methods know by those of skill in the art. Suitable examples include:
- **Colorimetric assays** - Iron is quantitated by measuring the intensity of the violet complex formed in the reaction between ferrozine and Fe²⁺ in acetate buffer at 562 nm. Thiourea or other chemicals can be added to complex contaminant metals such Cu²⁺, which can also bind with ferrozine and yield falsely elevated iron values. See Ceriotti et al.. Improved direct specific determination of serum iron and total iron-binding capacity Clin Chem. 1980. 26(2). 327-31, the contents of which are incorporated herein by reference.
- **Inductively Coupled Plasma Atomic Emission Spectroscopy (ICP-AES)** - is an emission spectroscopy technique that quantifies the mass percentage of metals in a sample. ICP-AES is based on the excitation of metal atoms/ions in the sample using a plasma (an ionized gas consisting of positive ions and free electrons) and analyzing the emission wavelength of the electromagnetic radiation, which is typical of that particular metal. Even though the technique is a standard analytical technique within common general knowledge of the skilled person, further information on ICP-AES can be found in Manley et al., Simultaneous Cu-, Fe-, and Zn-specific detection of metalloproteins contained in rabbit plasma by size-exclusion chromatography-inductively coupled plasma atomic emission spectroscopy. J Biol Inorg Chem. 2009, 14, 61-74, the contents of which are incorporated herein by reference.

The preferred method of determining the iron content of a sample for the purposes of the present invention is ICP-AES. The iron saturation of transferrin, for example, is then calculated based on the transferrin protein concentration, total iron content of the sample, and the fact that wild type transferrin has two iron-binding sites. Wild type human transferrin (molecular weight 79,750) can bind two iron atoms such that a sample containing 1 g of transferrin would be 100% saturated by 1.4 mg of iron.

At the time of writing, transferrin has not been authorized as a pharmaceutical in any major jurisdiction worldwide. As such, pharmacopoeial monographs do not exist for transferrin. Further information on the physical properties of transferrin, such as iron saturation can be obtained from the main reference text consulted by the skilled person; See L von Bonsdorff et al., Transferrin. Ch 21, pg 301-310, Production of Plasma Proteins for Therapeutic Use, Eds. J. Bertolini et al., Wiley, 2013 [Print ISBN:9780470924310 |Online ISBN:9781118356807], the contents of which are incorporated herein by reference and would be deemed to be within the common general knowledge of the skilled person.

In a further embodiment, the present invention includes mutant forms of transferrin and/or lactoferrin that maintain their structure but prevent the protein binding iron to one or the other of the iron-biding domains, e.g. the N-lobe, the C-lobe, or a combination thereof.

In one particular embodiment, transferrin mutants combinable with alpha-1 antitrypsin within the scope of the present invention include, but are not limited to:
i) Y188F mutant N lobe (SEQ ID NO: 4);
ii) Y95F/Y188F mutant N lobe (SEQ ID NO: 5);
iii) Y426F/Y517F mutant C lobe (SEQ ID NO: 6);
iv) Y95F/Y188F/Y517F mutant N and C lobes (SEQ ID NO: 7); and
v) Y95F/Y188F/Y426F/Y517F mutant N and C lobes (SEQ ID NO: 8).

In one embodiment of the invention, the transferrin and lactoferrin proteins combined with alpha1-antitrypsin in the present invention are fusion proteins having an improved *in-vivo* half-life, in which:
- the wild type (mammalian, preferably human) transferrin or lactoferrin protein is fused to a fusion partner selected from an immunoglobulin Fc domain and albumin; or
- a mutant transferrin or lactoferrin protein within the scope of the method of the present invention is fused to a fusion partner selected from an immunoglobulin Fc domain and albumin.

In one embodiment, the preferred fusion partner is an immunoglobulin Fc domain. For example, the immunoglobulin Fc domain may comprise at least a portion of a constant heavy immunoglobulin domain. The constant heavy immunoglobulin domain is preferably an Fc fragment comprising the CH2 and CH3 domain and, optionally, at least a part of the hinge region. The immunoglobulin Fc domain may be an IgG, IgM, IgD, IgA or IgE immunoglobulin Fc domain, or a modified immunoglobulin Fc domain derived therefrom. Preferably, the immunoglobulin Fc domain comprises at least a portion of a constant IgG immunoglobulin Fc domain. The IgG immunoglobulin Fc domain may be selected from IgG1, IgG2, IgG3 or IgG4 Fc domains, or modified Fc domains thereof.

In one embodiment, the fusion protein may comprise transferrin fused to an IgG1 Fc domain. In one embodiment, the fusion protein may comprise a transferrin mutant fused to an IgG1 Fc domain. For example, the fusion protein may comprise a Y95F/Y188F/Y426F/Y517F transferrin mutant and IgG1 Fc domain set forth in SEQ ID NO: 9.

It should be appreciated by those skilled in the art that the specific embodiments disclosed within this section (Combination Therapy) should not be read in isolation, and that the present specification intends for these embodiments to be disclosed in combination with other embodiments as opposed to being disclosed individually. For example, where the context does not imply otherwise, each of the embodiments disclosed in this section (Combination Therapy) is to be read as being explicitly combined with each of the embodiments in sections Treatment of the Invention, Neural Cell Injury, Dosing & Administration, and Pharmaceutical Composition of the Invention, or any permutation of 2 or more of the embodiments disclosed therein.

### Brief Description of the Drawings

Additional features and advantages of the present invention will be made clearer in the appended drawings, in which:
**Figure 1** provides images of brain staining in a mouse model of perinatal hypoxia-ischemia, wherein the mice have been subjected to sham, vehicle and a treatment regimen of the present invention;
**Figures 2A & 2B** plot the performance of mice subjected to conditions inducing hypoxia-ischemia in widely utilised cognition tests (NOLT and T-maze); mice were subjected to sham, vehicle and a treatment regimen of the present invention;
**Figures 3A to 3C** plot the performance of mice subjected to conditions inducing hypoxia-ischemia in widely utilised motor function tests (rotarod and balance-beam); mice were subjected to sham, vehicle and a treatment regimen of the present invention;
**Figures 4A & 4B** illustrate the effect of a treatment regimen of A1AT in accordance with the present invention on the levels of caspase-3 in mice subjected to conditions inducing hypoxia-ischemia;
**Figures 5A & 5B** illustrate the effect of a treatment regimen of A1AT in accordance with the present invention on the levels of GFAP and Iba1 in mice subjected to conditions inducing hypoxia-ischemia;
**Figures 6A & 6B** illustrate the effect of a treatment regimen of A1AT in accordance with the present invention on the levels of proinflammatory biomarkers in mice subjected to conditions inducing hypoxia-ischemia;
**Figure 7** illustrate the effect of a treatment regimen of A1AT in accordance with the present invention on the levels of myelin basic protein in mice subjected to conditions inducing hypoxia-ischemia;
**Figure 8** plots the performance of mice subjected to a spinal cord injury in the widely utilised BMS locomotive test; mice were subjected to a vehicle and a treatment regimen of the present invention;
**Figure 9** plots the performance of mice subjected to a spinal cord injury in the widely utilised plantar algesimetry test; mice were subjected to a vehicle and a treatment regimen of the present invention;
**Figure 10** illustrates the effect of a treatment regimen of A1AT in accordance with the present invention on the levels of NeuN in mice subjected to spinal cord injury;
**Figures 11(A)** - **(D)** illustrate the effect of a treatment regimen of A1AT in accordance with the present invention on the levels of GFAP (A), Iba-1 (B), MBP (C) and O4 (D) in mice subjected to spinal cord injury;
**Figures 12(A) & (B)** illustrate the effect of a treatment regimen of A1AT in accordance with the present invention on the levels of vascular regeneration in mice subjected to spinal cord injury; and
**Figures 13(A) & (B)** illustrate the effect of a treatment regimen of A1AT in accordance with the present invention on the prevention of glial scar formation in mice following spinal cord injury.

### Detailed Examples of the Invention

It should be readily apparent to one of ordinary skill in the art that the examples disclosed herein below represent generalised examples only, and that other arrangements and methods capable of reproducing the invention are possible and are embraced by the present invention.

### Alpha-1 Antitrypsin Therapy in a Perinatal Hypoxia-Ischemia (HI) Mouse Model

### Example 1: HI Induction in Mice

In C57/BL6 mice, at the postnatal age of 8 days (P8), ligation of the left common carotid artery was performed for 1 hour and the animals were subsequently placed under hypoxic conditions (90% N₂ and 10% O₂) for 50 minutes so as to induce HI within the mice. Following termination of the hypoxic conditions, the animal is placed in a cage for recovery.

Six hours after inducing HI, intraperitoneal administration of different doses of human alpha-1 antitrypsin (hA1AT) were delivered to the mice: 25, 50, 75, 100, 150 and 200 mg/Kg. Individual mice received the same dose daily over a 7 day period, e.g. a mouse that received 50 mg/Kg on day 1 continued to receive the same 50 mg/Kg dose daily over a 7 day dosing period. Where necessary, at the end of the 7 days the mice were sacrificed to perform subsequent biomarker analyses.

### Example 2: Effect of hA1AT dose on Brain Lesion Volume

Hematoxylin-eosin (H-E) and 2,3,5-triphenyltetrazolium chloride (TTC) staining were performed to visualize the correlation between brain injury/lesion volume and the conditions (vehicle [saline], treatment, or sham) that the mice were subject to. Staining is utilised to measure the volume of a lesion in the cortex, hippocampus and striatum following HI induction in the mice. Mice were perfused with heparinized saline followed by 4% paraformaldehyde, and the brain was processed to obtain cryostat sections.

The results are presented in **Table 1.** Values are expressed as % with respect to the volume measured under sham conditions as mean ± SEM (n=4) and a one-way ANOVA with a Bonferroni's *post hoc* test was performed. As shown in **Table 1**, perinatal HI causes a reduction of cortical, hippocampal and striatal brain volume greater than 50% in the group of mice treated with vehicle only.

Mice treated with hA1AT following the induction of HI show no significant differences between the 25 mg/Kg dose and the vehicle (**Table 1**). Doses upwards of 50 mg/Kg provide an observable neuroprotective effect and significant increases in volume of healthy cells in the cortex and hippocampus are noted. The efficacy of hA1AT appears to plateau at the 100mg/Kg - very little additional benefit is seen with doses greater than 100 mg/Kg.

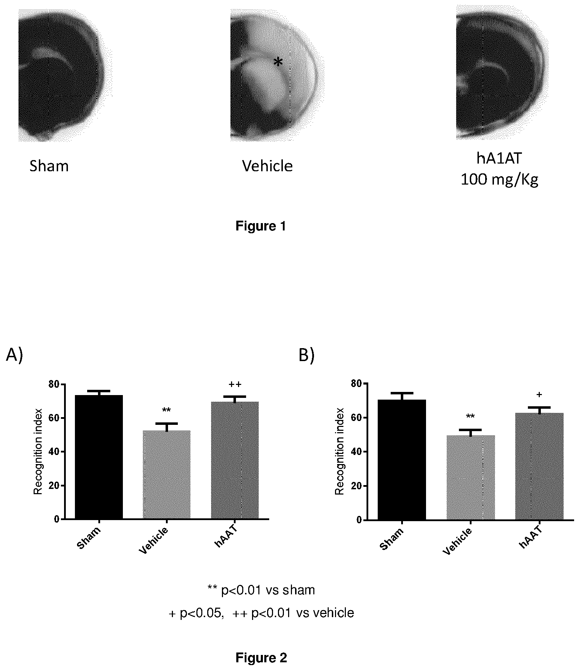

Representative images of brain TTC staining in sham condition, vehicle/HI and hA1AT treatment at 100 mg/Kg are shown in **Figure 1****.** Mice treated with the vehicle show a considerable loss of TTC staining (lighter region denoted with asterisk) with respect to the sham, characteristic of the loss of volume due to the HI induced injury. The brain slice from mice treated with 100 mg/Kg hA1AT shows a high volume of TTC staining indicating that 100 mg/Kg of hA1AT successfully prevented brain lesions of considerable volume forming.

**Table 1**

| CONDITIONS | CORTEX | HIPPOCAMPUS | STRIATUM |
|---|---|---|---|
| Sham | 100±3.3 | 100±4.5 | 100±10.2 |
| Vehicle/HI | 39±3.3*** | 41±3.5*** | 49±5.2*** |
| 25 mg/Kg | 40±7.3 | 45.7±2.9 | 53.8±3 |
| 50 mg/Kg | 51.1±3.9⁺ | 53.3±3.7⁺ | 55.4±3.3 |
| 75 mg/Kg | 65.8±6.3⁺⁺ | 70.4±35.5⁺⁺ | 69.3±6.2⁺ |
| 100 mg/Kg | 80.2±5.3⁺⁺⁺ | 82.8±6.5⁺⁺⁺ | 85.8±7.1⁺⁺⁺ |
| 150 mg/Kg | 81.4±4.3⁺⁺⁺ | 80.5±5.9⁺⁺⁺ | 83.5±7.1⁺⁺⁺ |
| 200 mg/Kg | 83.4±5.1⁺⁺⁺ | 79.6±5.7⁺⁺⁺ | 85.7±6.7⁺⁺⁺ |

| | | | |
|---|---|---|---|
| *** p<0.001 vs sham; ⁺p<0.05, ⁺⁺p<0.01, and ⁺⁺⁺p<0.001 vs vehicle. | | | |

### Example 3: Improvements in Cognition in HI Mice Treated with hA1AT

After the ischemic injury to the 8-day-old C57/BL6 mice outlined in **Example 1**, the mice were treated with intraperitoneal administration of hA1AT at a dose of 100 mg/Kg/day every day for one week, and subsequent bi-weekly administration of the same 100 mg/Kg dose for 6 weeks. Once the dosing period was finished, cognitive tests were performed followed by motor tests (*vide infra*) on the subsequent day. The mice were subjected to two cognitive tests: NOLT (novel object location test) and T-maze (T-shaped arm). In the first test, the animal is allowed to explore two objects in an enclosed area for 2 to 3 minutes. After 24 hours the long-term memory is evaluated. When the mice are positioned in the same enclosed area, one of the objects is relocated to a different position in the enclosed area. Mice with good memories spend more time exploring the relocated object. The results of the test are expressed in terms of a recognition index: higher recognition indices equate with better long term memory and recognition of the relocated object.

The cognition test results in **Figure 2** are expressed in % as the recognition index of the new object (A) or the new maze-arm (B) with respect to the old object (A) or the old arm (B). A mean ± SEM (n=6) and one-way ANOVA with a Bonferroni's post hoc test were performed.

From **Figure 2A** it can be seen that the sham group recorded a recognition index of 73%±3. In contrast, the injured animals (vehicle) recorded a recognition index of 52%±5, indicating that they explore both objects equally and correlates to deficits in memory processes. Gratifyingly, animals treated with the 100 mg/Kg hA1AT dosing regimen demonstrate a statistically significant higher recognition index than the vehicle (69%±4). Importantly, the recognition index for the hA1AT treated mice approximates very closely to that of the sham group (**Figure 2A**).

In the T-maze test the animals are allowed to explore a T-shaped arm but with one of the arms closed. After 8 hours, the arm is opened and the animals are exposed to the maze again. Mice with good memories spend more time exploring the newly opened arm for longer (index of recognition). The results of the T-maze test are presented in **Figure 2B****.** The untreated vehicle group returned a recognition index of 49%±4 compared to the sham group with a recognition index of 70%±4.5. This result is consistent with the vehicle group having suffered neural damage. Similar to the NOLT test, the group treated with hA1AT dosing regimen recorded a statistically significant increase in the recognition index (62%±4) with respect to the vehicle.

### Example 4: Improvements in Motor Function in HI Mice Treated with hA1AT

After the ischemic injury to the 8-day-old C57/BL6 mice as per **Example 1**, the mice were treated with intraperitoneal administration of hA1AT at a dose of 100 mg/Kg/day every day for one week, and subsequent bi-weekly administration of the same 100 mg/Kg dose for 6 weeks. Once the dosing period was finished, motor function tests were performed on the subsequent day.

Motor function of the mice was evaluated in two tests: the rotarod and the balance-beam. The rotarod test consists of a rotating cylinder at two different speeds (16 and 24 rpm) and the number of times in a minute that a mouse falls from the cylinder is calculated. The higher the number of falls, the lower the animal's motor coordination capacity.

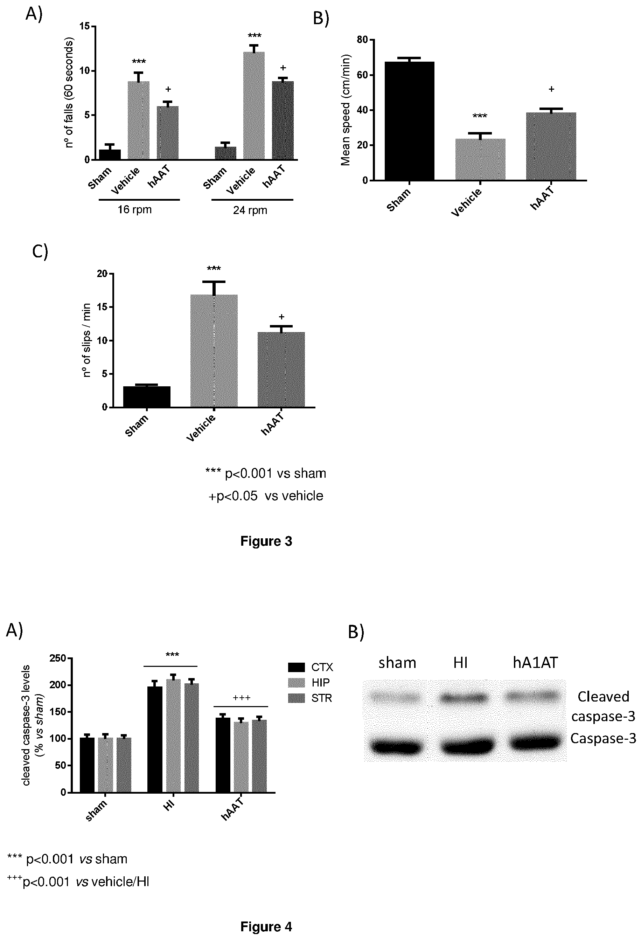

The results of the motor function tests are plotted in **Figure 3** and measure, during a 1 minute time period; the number of falls from the rotarod (A), distance travelled on the balance beam in cm (B) and the number of slips from the balance beam (C). The mean ± SEM (n=6) and one-way ANOVA with Bonferroni's *post hoc* test were performed.

After 6 weeks, following the hA1AT dosing regimen, the rotarod (**Figure 3A**) and balance-beam (**Figures 3B and 3C**) tests were performed on sham, vehicle/HI and hA1AT treated animals. Animals expected to have significant neural injuries (vehicle group) show a high number of falls at both 16 rpm and 24 rpm speeds, and the results are tabulated in **Table 2**. Consistent with other results thus far, the hA1AT treated group has statistically significant lower number of falls compared to the vehicle group.

**Table 2**

| | Falls per minute 16 rpm | Falls per minute 24 rpm |
|---|---|---|
| Sham | 1±1 | 1.3±0.6 |
| Vehicle/HI | 8.7±1.1 | 12±0.9 |
| hA1AT | 5.9±0.6 | 8.7±0.5 |

Similar results were observed with the balance beam test. In this test, the animal goes over a cylinder and the distance travelled in one minute and the number of animal slips are calculated. The results are shown in **Figures 3B and 3C** and tabulated below in **Table 3**.

**Table 3**

| | cm/min | slips per minute |
|---|---|---|
| Sham | 67±3 | 3±0.4 |
| Vehicle/HI | 23±3.9 | 16.7±2.1 |
| hA1AT | 38±2.8 | 11.1±1.1 |

Injured animals walk across at a slower speed than sham group (**Figure 3B**) and have a greater number of paw slips (**Figure 3C**). Significantly, animals treated with hA1AT demonstrate improved motor function, showing a greater distance travelled and a fewer number of times that one paw slips with respect to the vehicle.

Importantly mice treated with hA1AT showed significant improvements in two very different brain functions controlled by different brain areas; cognition and motor function.

### Example 5: Biomarker Levels in HI Mice Treated with hA1AT

All biomarker studies were performed on mice subjected to the conditions outlined in **Example 1**.

### Caspase-3: Apoptosis

HI induction produces the activation of necrotic death initially, and apoptotic death subsequently. The ability of hA1AT to modulate apoptosis was determined by measuring the levels of the active fragment of caspase-3. **Figure 4** shows the results of the analysis of active caspase-3 by Western Blot in the 3 different brain areas. HI induces an increase of the active fragment of caspase-3 in all three areas (see **Figure 4A****:** cortex: 195%±12; hippocampus: 209%±11; striatum: 201%±10). In **Figure 4A**, the results are expressed as % with respect to the sham group, and as mean ± SEM (n=6). A one-way ANOVA with a Bonferroni's post hoc test has been performed.

Daily administration of hA1AT for 7-days at 100 mg/Kg/day reduces caspase-3 activation significantly, with no significant differences observed between treatment and sham (results are expressed in terms of % with respect to the sham group: cortex: 137%±9; hippocampus: 129%±9; striatum: 133%±8). These results indicate hA1AT at a 100 mg/Kg dose markedly reduced apoptotic death in the three brain areas to levels that are not significantly different with respect to sham group. Representative immunoblots from the cortex area are shown in **Figure 4B**.

### Glial fibrillary acidic protein (GFAP) & Ionized calcium binding adaptor molecule 1 (Iba1): Glial Cell Reactivity

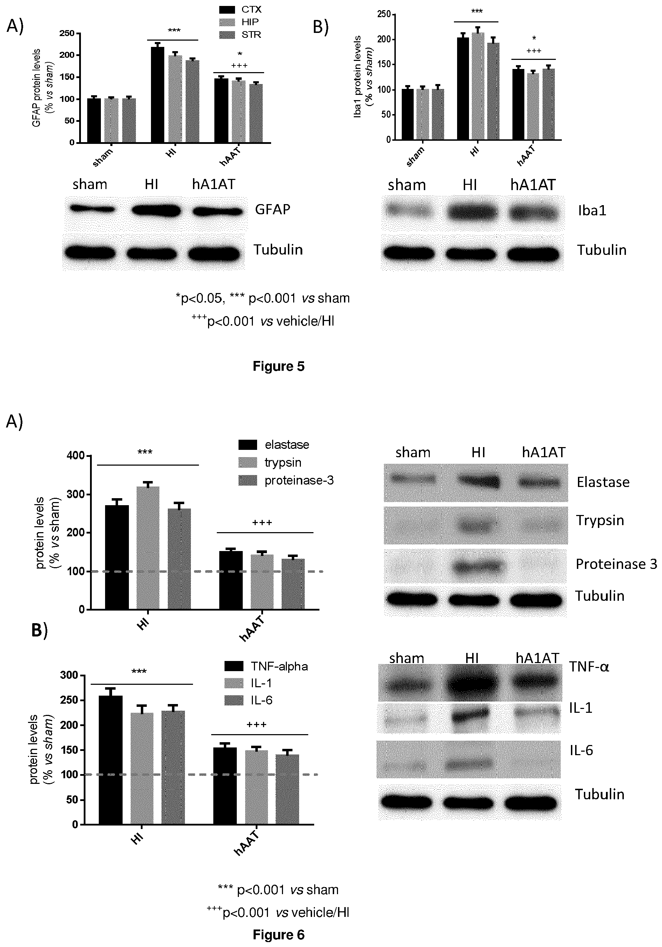

Glial reactivity causes considerable neuronal toxicity. Astrocyte reactivity was determined by measuring GFAP levels by Western blot in the three brain areas in mice: sham, vehicle/HI and treated with 100 mg/Kg/day of hA1AT for 7 days after HI. GFAP is a protein from the astrocyte cytoskeleton, and its levels increase when it is reactive. **Figure 5A** illustrates that HI induction results in an increase in GFAP levels in all three brain areas (cortex: 217%±11; hippocampus: 198%±10; striatum: 187%±6). The results are expressed in terms of % with respect to the sham, and as mean ± SEM (n=6). A one-way ANOVA with a Bonferroni's post hoc test was performed. The animals treated with hA1AT express a statistically significant reduction compared to the group of mice exposed to HI in the three brain areas (145%±7; hippocampus: 141%±6; striatum: 133%±7).

Similar results are obtained by measuring the protein levels of Iba1, a specific protein of microglial cells. **Figure 5B** illustrates that HI induction results in an increase in Iba1 levels in all three brain areas (cortex: vehicle 202%±11 - hA1AT 139%±8; hippocampus: vehicle 212%±13 - hA1AT 131%±7; striatum: vehicle 195%±10 - hA1AT 140%±6). The results are expressed in terms of % with respect to the sham, and as mean ± SEM (n=6). A one-way ANOVA with a Bonferroni's post hoc test was performed.

Accordingly, we can conclude that HI induction produces astrocytic and microglial reactivity in the three brain areas, and that the administration of hA1AT in accordance with the treatment regimen of the present invention significantly reduces this reactivity.

### Elastase, Trypsin, Proteinase-3, TNFα, IL-1 and IL-6: Pro-inflammatory proteases and cytokines

The inflammatory process is very important to the development of tissue injury in HI. Inflammation was assessed by measuring elastase, trypsin and proteinase-3 levels by Western blot in mice: sham, vehicle/HI and treated with 100 mg/Kg/day of hA1AT for 7 days after HI. The levels in the cerebral cortex are represented in **Figure 6A****.** Results are expressed in terms of % with respect to the sham condition (dashed line), and as mean ± SEM (n=6). A one-way ANOVA with a Bonferroni's post hoc test was performed. It was observed that HI induction produces an increase in the 3 proteases with respect to the sham condition (dashed line; elastase: 269%±18; trypsin: 317%±15; proteinase-3: 260%±18). Importantly, the administration of hA1AT in accordance with the present invention significantly reduces the concentration of these proteases. A statistically significant reduction of the 3 protease levels in the cerebral cortex of the group of mice treated with hA1AT was found (elastase: 149%±9.1; trypsin: 141%±10.5; proteinase-3: 130%±11.1). Similar results have been obtained in the hippocampus and striatum (figure not shown).

The levels of 3 proinflammatory cytokines were also studied (TNFα, IL-1 and IL-6) by Western blot in mice: sham, vehicle/HI and treated with 100 mg/Kg/day of hA1AT for 7 days after HI. The obtained results present the same trend observed with proteases levels. The levels in the cerebral cortex are represented in **Figure 6B****.** Results are expressed in terms of % with respect to the sham condition (dashed line), and as mean ± SEM (n=6). A one-way ANOVA with a Bonferroni's post hoc test was performed. It is observed that HI induction produces an increase in the 3 cytokines with respect to the sham condition: (dashed line: TNFα: vehicle 257%±17 - hA1AT 153%±10.7; IL-1: vehicle 223%±16 - hA1AT 147%±9.9; IL-6: vehicle 227%±13 - hA1AT 139%±11).

Similar results have been obtained in the hippocampus and striatum (figure not shown). These results indicate that hA1AT dosing of the present invention can prevent the inflammatory process induced by HI.

### Myelin Basic Protein (MBP): Brain White Matter Levels

During ischemic process, both white and grey brain matter deteriorate. Accordingly, therapeutics should have a neuroprotective effect that extends to the preservation of both white and grey matter.

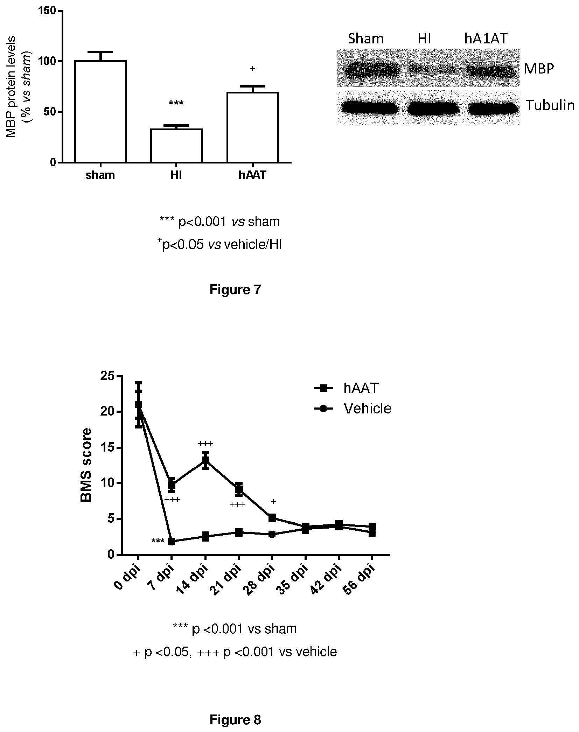

White matter preservation was assessed by measuring MBP (Myelin Basic Protein), a protein that belongs to myelin, in the subcortical region of the brain. The levels were assessed by Western blot in mice: sham, vehicle/HI and treated with 100 mg/Kg/day of hA1AT for 7 days after HI. The levels of MBP in the subcortical region are illustrated in **Figure 7****.** Results are expressed in terms of % with respect to the sham condition. A mean ± SEM (n=6) and one-way ANOVA with a Bonferroni's post hoc test was performed.

MBP levels are statistically lower in the brain of HI-induced mice showing white matter deterioration (in % with respect to sham group: 33%±4). Significantly, an increase in MBP levels is observed in the group of animals treated with hA1AT compared to HI animals (in % with respect to sham group: 71%±6); demonstrating that hA1AT preserves white matter during HI conditions. A representative immunoblot is also shown.

### Alpha-1 Antitrypsin Therapy in Acute Spinal Cord Injury (SCI)

### Example 8: Induction of a SCI in Mice

Spinal cord injury was induced in mice (Balb/C strain) by dropping a weight on a mouse's spinal cord. A 5 g weight was dropped from a launch distance of 10 mm on to the mouse's spinal cord. Experimentation had shown that the latter conditions resulted in an injury/lesion volume that did not exceed 50% of the spinal medullar volume at the epicentre of injury induction, as determined by hematoxylin-eosin (H-E) staining.

### Example 9: Evaluation of the Effect of hA1AT Doses on Recovery from Traumatic Spinal Cord Injury

hA1AT was administered to the injured mice at different doses (25, 50, 75, 100, 150, and 200 mg/Kg) via intraperitoneal administration. The first dose was administered after three hours post-injury, to allow the animal recovery after the operation, and was repeated daily for 14 days. Functional tests were performed every week until week 8, and morphological and biochemical tests were performed at the end of treatment (2 weeks) and at 8 weeks post-injury.

To determine the most effective dose of hA1AT, the area of the spinal cord injury was measured using HE staining. The H-E stain is performed and the area that has not been stained is analyzed in relation to the total medullary surface. The results in **Table 4** are expressed as the % of the area occupied by the lesion with respect to the total medullary area. A mean ± SEM (n = 4) and a one-way ANOVA analysis were performed with a Bonferroni's post hoc test. As seen from the vehicle entry in **Table 4**, the spinal cord injury procedure produces a viable cell matter loss of 43.8% at 14 days post injury (dpi). At 56 dpi, the injury area does not increase, being 45.3% at 56 dpi.

At 14 dpi, hA1AT administration substantially reduces the injury area at doses of 100 mg/Kg and greater. At doses higher than 100 mg/Kg the reduction of the lesion volume to is not statistically significant with respect to the dose of 100 mg/Kg. Lower doses (25 and 50 mg/Kg) do not produce any effect on the lesion volume with respect to the vehicle at 14 dpi. The dose of 75 mg/Kg, although it induces a reduction in the volume of the lesion, this is not statistically significant with respect to the vehicle.

This protective effect of hA1AT at 56 dpi is less pronounced. Doses of 25 to 75 mg/Kg do not induce any reduction in the lesion volume with respect to the vehicle. For doses from 100 to 200 mg/Kg, there is an observable decrease in the lesion volume. The data trends indicate that hA1AT has a protective effect on spinal cord injury at doses of about 100 mg/Kg or greater, and that this protective effect is best potentiated by continued administration hA1AT as part of a multiple dosage regimen.

**Table 4**

| CONDITIONS | 14 DPI* | 28 DPI | 56 DPI |
|---|---|---|---|
| Vehicle [saline] | 43.8±4.2 | 46.5±5.7 | 45.3±4.9 |
| 25 mg/Kg | 44.9±7.2 | - | 43.9±6.2 |
| 50 mg/Kg | 40.3±6.2 | - | 47.1 ±8.2 |
| 75 mg/Kg | 35.7±4.9 | - | 40.3±5.8 |
| 100 mg/Kg | 22.1 ±5.2++ | 28.4±4.1 | 34.4±6.2 |
| 150 mg/Kg | 20.2±4.2++ | - | 35.5±4.5 |
| 200 mg/Kg | 18.9±4.0++ | - | 33.1 ±5.8 |

| | | | |
|---|---|---|---|
| * DPI = Days Post Injury ++ p <0.01 vs vehicle | | | |

### Example 10: Improvements in Motor Function in SCIMice Treated with hA1AT

The Basso Mouse Scale (BMS) is a well-known locomotive test that analyses 6 locomotor parameters in a single test. These 6 parameters are combined to create a scale giving a value (the higher the value, the greater the locomotor capacity of the animal). This test is particularly applicable for measuring locomotor failure caused by spinal cord injury in the Balb/C strain. For further particulars, see Basso et al.. J. Neutrauma 23 (2006) 635-659.

Spinal Cord Injury was induced in Balb/C strain mice in accordance with the procedure outlined in **Example 8.** The mice were treated with intraperitoneal administration of hA1AT (100 mg/Kg). Every 7 dpi (days post injury), the mice were assessed using the BMS locomotive test. The results are plotted in **Figure 8** and expressed according to the BMS standardized scale. Sham animals had a mean value of 20 (not shown). A mean ± SEM (n = 6) and a one-way ANOVA analysis were performed with a Bonferroni's *post hoc* test.

Both groups of mice (vehicle and hA1AT) had a BMS locomotor value of 20 prior to injury, confirming good locomotor status and that there are no significant differences between both groups. Subsequent spinal cord injury manifests in a drastic reduction in the mice BMS locomotor score (See **Figure 8**, 7 dpi; value of the BMS scale: 1.8% ± 0.3). The diminution in locomotor capacity (BMS score < 4) remained throughout the entire observation period, up to 56 dpi.

Daily intraperitoneal administration of hA1AT 100 mg/Kg, for 14 days, significantly improved the motor function/BMS score of the mice at both 7 (9.7% ± 0.9) and 14 dpi (13.2% ± 1.1) time points with respect to the vehicle group. The magnitude of the improvement reduced once treatment with hA1AT had stopped. However, at 21 and 28 dpi statistically significant improvements in BMS score were still observed in the hA1AT treatment arm (**Figure 8**; 21 dpi - 9.1% ± 0.8; 28 dpi - 5.1% ± 0.4).

### Example 11: Reduction in Neuropathic Pain in SCI Mice Treated with hA1AT

In approximately 50% of spinal cord injury cases, neuropathic pain develops, a factor that considerably reduces the quality of life and has been shown to be the cause of a significant number of suicides in spinal cord injury patients. To determine the effect of hA1AT in reducing neuropathic pain, the mice were assessed using plantar algesimetry. The latter involves projecting a laser on to the sole of the animal's paw and measuring the time taken to remove the paw; a maximum time limit is set to avoid causing injury to the mouse. Quicker withdrawal of the paw indicates increased sensitisation of the mouse to pain on account of suffering with neuropathic pain.

Spinal Cord Injury was induced in Balb/C strain mice in accordance with the procedure outlined in **Example 8.** The mice were treated with intraperitoneal administration of hA1AT (100 mg/Kg) in accordance with the 14 day dosage regimen outlined in **Example 9**. Every 7 dpi (days post injury), the mice were assessed using the plantar algesimetry test discussed above. Results are expressed in paw withdrawal time (seconds). Sham animals presented a mean value of 20 seconds (not shown). A mean ± SEM (n = 6) and a one-way ANOVA analysis were performed with a Bonferroni's *post hoc* test.

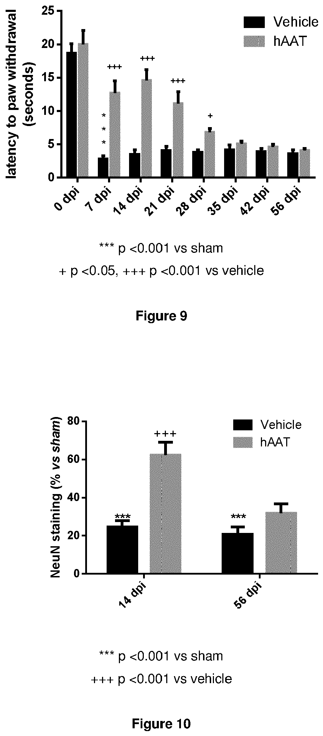

Plantar algesimetry assessment was performed in both groups (vehicle and hA1AT) before injury, confirming that there were no significant differences between the two groups (0 dpi). Subsequent spinal cord injury manifests in increased pain sensitization within the mice. With reference to **Figure 9**, at 7 dpi, the vehicle group exhibited a very fast paw withdrawal indicative of neuropathic pain (7 dpi; 2.8 ± 0.5; 14 dpi; 3.5 ± 0.7; 21 dpi; 4.1 ± 0.6). hA1AT treated mice presented a slower withdrawal, this being statistically significant with respect to the vehicle group up to 28 dpi. The results validate the hypothesis that hA1AT successfully reduces neuropathic pain in mice subjected to spinal cord injuries.

In summary, these results demonstrate the therapeutic capacity of hA1AT in locomotor recovery but also in the prevention of the onset of neuropathic pain. This effect is pronounced during early stages of the treatment (up to 14 dpi).

### Example 12: Biomarker analysis in SCI mice treated with hA1AT

The effects of a 100 mg/Kg dose of hA1AT in SCI mice on the levels of a number of different biomarkers was assessed. **Figure 10** illustrates the effect of hA1AT on the levels of NeuN, a neuronal marker, to determine the neuroprotective effect on the spinal cord 14 days after spinal cord injury. After the spinal cord injury induction, the animals were treated with intraperitoneal hA1AT (100 mg/Kg). At 14 and 56 dpi, a NeuN stain is performed on spinal cord sections and the positive areas are measured. The results are expressed in % with respect to the sham (100% NeuN staining). A mean ± SEM (n = 4) and a one-way ANOVA analysis were performed with a Bonferroni's *post hoc* test. The administration of hA1AT considerably reduces neuronal loss following spinal cord injury.

The effect of hA1AT administration on the spinal cord levels of glial reactivity [GFAP (astrocyte marker) and Iba-1 (microglial marker)] and the preservation of white matter in the spinal cord [MBP (protein from myelin), and O4 (marker of oligodendrocytes, cells responsible for producing myelin)] was determined by Western Blot. Tubulin protein levels are used as a control.

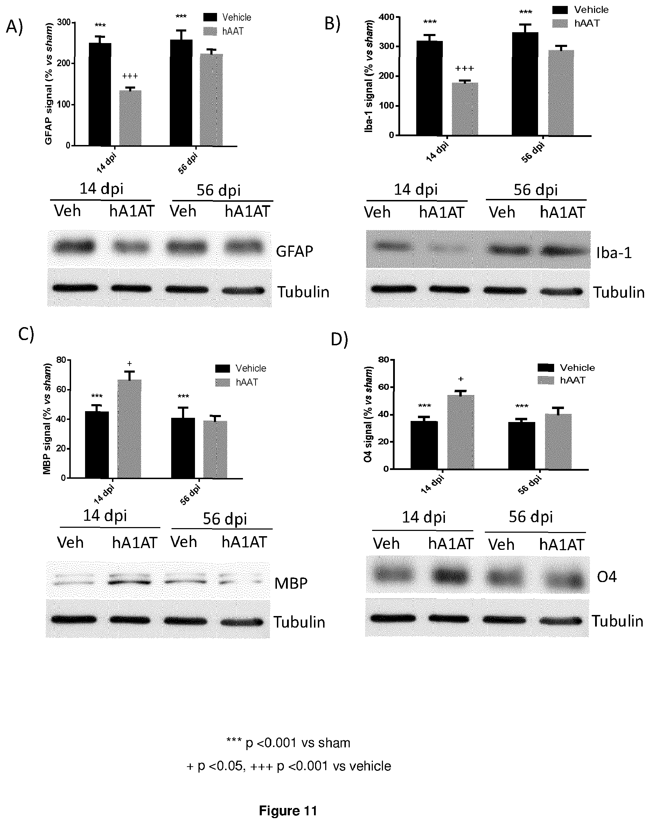

**Figure 11** illustrates the effect of hA1AT on GFAP (A), Iba-1 (B), MBP (C) and O4 (D). The results are expressed in % with respect to the sham condition (100 %). A mean ± SEM (n = 6) and a one-way ANOVA analysis were performed with a Bonferroni's *post hoc* test. Intraperitoneal hA1AT (100 mg/Kg) administration reduces glial reactivity induced by spinal cord injury and preserves elements of the white matter as shown in **Figure 11****.**

### Example 13: Evaluation of the Effect of hA1AT on Spinal Revascularization after SCI

Vascular regeneration and the dissolution of the glial scar are two key elements for nerve regeneration after a spinal cord injury that assist in a functional recovery. To assess the effect of hA1AT administration on these processes the levels of reca-1 (a marker of endothelial status), and collagen-IV (a marker of glial scar status) was determined.

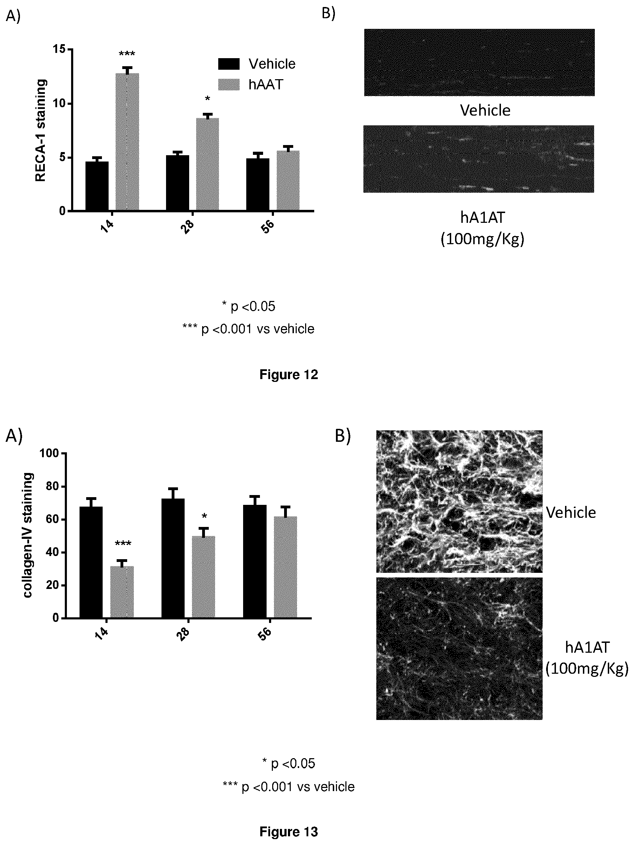

The injury and treatment protocol is per the procedure outlined in **Examples 8 & 9**. The animals have been injured by blunt trauma and subsequently treated with 100 mg/Kg of hA1AT for 14 days. The days post injury (dpi) selected to perform the analysis were: 14 dpi (subacute state and end of treatment), 28 dpi and 56 dpi (both intermediate-long post-injury state). For all cases, 4 animals per group were used. At 14, 28 and 56 dpi, a reca-1 stain is performed and the result obtained in each time period was analysed and plotted in **Figure 12(A)****.** The results are expressed as a mean ± SEM (n = 4). The sham group presents an average of 18% of reca-1 stained area (not shown). A one-way ANOVA analysis is performed with a Bonferroni's *post hoc* test. **Figure 12(B)** provides representative images of staining by reca-1 in vehicle/SCI group and group treated with hA1AT at 14 dpi (scale bar: 100 µM).

The reca-1 staining shows that the administration of hA1AT for 14 days is capable of statistically significant increased reca-1 levels with respect to the vehicle (**Figure 12(A)**; 14 dpi in absolute values of stained area: vehicle; 4.5% ± 0.5; hA1 AT; 12.7% ± 0.6). The magnitude of the effect is proportional to the levels of hA1AT in the patient's plasma; from results at 14, 28 and 56 dpi gradual declines in reca-1 staining are observable. The results indicate that hA1AT has the ability to promote vascular regeneration in the injured spinal cord area.

At 14, 28 and 56 dpi (days post injury), a collagen-IV stain is performed and the result obtained in each time period was analysed and plotted in **Figure 13(A)**. The results are expressed as a mean ± SEM (n = 4). The sham group does not show any staining because a glial scar is not formed (data not shown). One-way ANOVA analysis is performed with a Bonferroni's *post hoc* test. **Figure 13(B)** provides representative images of collagen-IV staining in the vehicle group and the group treated with hA1AT at 14 dpi (scale bar: 100 µM).

The collagen-IV staining in **Figure 13(A)** shows that the administration of hA1AT for 14 days is capable of a statistically significant decrease in collagen-IV levels with respect to the vehicle at 14 and 28 dpi, (14 dpi: vehicle; 67% ± 5.9; hA1AT; 31% ± 4.1; 28 dpi: vehicle; 72% ± 5.7; hA1AT; 47% ± 5.5). The effect diminishes once treatment stops. The results suggest that hA1AT has the ability to prevent glial scar formation following spinal cord injury.

**Figure 13(B)** shows two representative images at 14 dpi. In the vehicle group, there is a strong staining indicating a developed glial scar. In the hA1AT treated group, this staining is reduced, confirming the ability of hA1 AT to decrease glial scar formation.

### Sequences

The sequences referred to in the preceding text are outlined below in fasta format. In the event of a discrepancy between the sequence listed in this text and the corresponding sequence in the accompanying sequence listing, the sequence listed in this text shall be the prevailing sequence for the purposes of correcting an error.

SEQ ID NO: 1 Human Alpha-1 antitrypsin (UniProtKB Seq. No. P01009) protein sequence







SEQ ID NO: 2 Human Transferrin [UniProt Q06AH7] protein sequence
VPDKTVRWCAVSEHEATKCQSFRDHMKSVIPSDGPSVACVKKASYLDCIRAIAANEADAVTLDAGLVYD
AYLAPNNLKPVVAEFYGSKEDPQTFYYAVAVVKKDSGFQMNQLRGKKSCHTGLGRSAGWNIPIGLLYCD
LPEPRKPLEKAVANFFSGSCAPCADGTDFPQLCQLCPGCGCSTLNQYFGYSGAFKCLKDGAGDVAFVK
HSTIFENLANKADRDQYELLCLDNTRKPVDEYKDCHLAQVPSHTVVARSMGGKEDLIWELLNQAQEHFG
KDKSKEFQLFSSPHGKDLLFKDSAHGFLKVPPRMDAKMYLGYEYVTAIRNLREGTCPEAPTDECKPVKW
CALSHHERLKCDEWSVNSVGKIECVSAETTEDCIAKIMNGEADAMSLDGGFVYIAGKCGLVPVLAENYNK
SDNCEDTPEAGYFAVAVVKKSASDLTWDNLKGKKSCHTAVGRTAGWNIPMGLLYNKINHCRFDEFFSE
GCAPGSKKDSSLCKLCMGSGLNLCEPNNKEGYYGYTGAFRCLVEKGDVAFVKHQTVPQNTGGKNPDP
WAKNLNEKDYELLCLDGTRKPVEEYANCHLARAPNHAVVTRKDKEACVHKILRQQQHLFGSNVTDCSG
NFCLFRSETKDLLFRDDTVCLAKLHDRNTYEKYLGEEYVKAVGNLRKCSTSSLLEACTFRRP

SEQ ID NO: 3 Human Lactoferrin [UniProt P02788] protein sequence
GRRRRSVQWCTVSQPEATKCFQWQRNMRRVRGPPVSCIKRDSPIQCIQAIAENRADAVTLDGGFIYEA
GLAPYKLRPVAAEVYGTERQPRTHYYAVAVVKKGGSFQLNELQGLKSCHTGLRRTAGWNVPIGTLRPFL
NWTGPPEPIEAAVARFFSASCVPGADKGQFPNLCRLCAGTGENKCAFSSQEPYFSYSGAFKCLRDGAG
DVAFIRESTVFEDLSDEAERDEYELLCPDNTRKPVDKFKDCHLARVPSHAVVARSVNGKEDAIWNLLRQ
AQEKFGKDKSPKFQLFGSPSGQKDLLFKDSAIGFSRVPPRIDSGLYLGSGYFTAIQNLRKSEEEVAARRA
RVVWCAVGEQELRKCNQWSGLSEGSVTCSSASTTEDCIALVLKGEADAMSLDGGYVYTAGKCGLVPVL
AENYKSQQSSDPDPNCVDRPVEGYLAVAVVRRSDTSLTWNSVKGKKSCHTAVDRTAGWNIPMGLLFN
QTGSCKFDEYFSQSCAPGSDPRSNLCALCIGDEQGENKCVPNSNERYYGYTGAFRCLAENAGDVAFVK
DVTVLQNTDGNNNDAWAKDLKLADFALLCLDGKRKPVTEARSCHLAMAPNHAVVSRMDKVERLKQVLL
HQQAKFGRNGSDCPDKFCLFQSETKNLLFNDNTECLARLHGKTTYEKYLGPQYVAGITNLKKCSTSPLL
EACEFLRK

SEQ ID NO: 4 Y188F Transferrin N-lobe mutant protein
VPDKTVRWCAVSEHEATKCQSFRDHMKSVIPSDGPSVACVKKASYLDCIRAIAANEADAVTLDAGLVYD
AYLAPNNLKPVVAEFYGSKEDPQTFYYAVAVVKKDSGFQMNQLRGKKSCHTGLGRSAGWNIPIGLLYCD
LPEPRKPLEKAVANFFSGSCAPCADGTDFPQLCQLCPGCGCSTLNQYFGFSGAFKCLKDGAGDVAFVK
HSTIFENLANKADRDQYELLCLDNTRKPVDEYKDCHLAQVPSHTVVARSMGGKEDLIWELLNQAQEHFG
KDKSKEFQLFSSPHGKDLLFKDSAHGFLKVPPRMDAKMYLGYEYVTAIRNLREGTCPEAPTDECKPVKW
CALSHHERLKCDEWSVNSVGKIECVSAETTEDCIAKIMNGEADAMSLDGGFVYIAGKCGLVPVLAENYNK
SDNCEDTPEAGYFAVAVVKKSASDLTWDNLKGKKSCHTAVGRTAGWNIPMGLLYNKINHCRFDEFFSE
GCAPGSKKDSSLCKLCMGSGLNLCEPNNKEGYYGYTGAFRCLVEKGDVAFVKHQTVPQNTGGKNPDP
WAKNLNEKDYELLCLDGTRKPVEEYANCHLARAPNHAVVTRKDKEACVHKILRQQQHLFGSNVTDCSG
NFCLFRSETKDLLFRDDTVCLAKLHDRNTYEKYLGEEYVKAVGNLRKCSTSSLLEACTFRRP

SEQ ID 5 Y95F/Y188F Transferrin N-lobe mutant protein
VPDKTVRWCAVSEHEATKCQSFRDHMKSVIPSDGPSVACVKKASYLDCIRAIAANEADAVTLDAGLVYD
AYLAPNNLKPVVAEFYGSKEDPQTFFYAVAVVKKDSGFQMNQLRGKKSCHTGLGRSAGWNIPIGLLYCD
LPEPRKPLEKAVANFFSGSCAPCADGTDFPQLCQLCPGCGCSTLNQYFGFSGAFKCLKDGAGDVAFVK
HSTIFENLANKADRDQYELLCLDNTRKPVDEYKDCHLAQVPSHTVVARSMGGKEDLIWELLNQAQEHFG

KDKSKEFQLFSSPHGKDLLFKDSAHGFLKVPPRMDAKMYLGYEYVTAIRNLREGTCPEAPTDECKPVKW
CALSHHERLKCDEWSVNSVGKIECVSAETTEDCIAKIMNGEADAMSLDGGFVYIAGKCGLVPVLAENYNK
SDNCEDTPEAGYFAVAVVKKSASDLTWDNLKGKKSCHTAVGRTAGWNIPMGLLYNKINHCRFDEFFSE
GCAPGSKKDSSLCKLCMGSGLNLCEPNNKEGYYGYTGAFRCLVEKGDVAFVKHQTVPQNTGGKNPDP
WAKNLNEKDYELLCLDGTRKPVEEYANCHLARAPNHAVVTRKDKEACVHKILRQQQHLFGSNVTDCSG
NFCLFRSETKDLLFRDDTVCLAKLHDRNTYEKYLGEEYVKAVGNLRKCSTSSLLEACTFRRP

SEQ ID NO: 6 Y426F/Y517F Transferrin C-lobe mutant protein
VPDKTVRWCAVSEHEATKCQSFRDHMKSVIPSDGPSVACVKKASYLDCIRAIAANEADAVTLDAGLVYD
AYLAPNNLKPVVAEFYGSKEDPQTFYYAVAVVKKDSGFQMNQLRGKKSCHTGLGRSAGWNIPIGLLYCD
LPEPRKPLEKAVANFFSGSCAPCADGTDFPQLCQLCPGCGCSTLNQYFGYSGAFKCLKDGAGDVAFVK
HSTIFENLANKADRDQYELLCLDNTRKPVDEYKDCHLAQVPSHTVVARSMGGKEDLIWELLNQAQEHFG
KDKSKEFQLFSSPHGKDLLFKDSAHGFLKVPPRMDAKMYLGYEYVTAIRNLREGTCPEAPTDECKPVKW
CALSHHERLKCDEWSVNSVGKIECVSAETTEDCIAKIMNGEADAMSLDGGFVYIAGKCGLVPVLAENYNK
SDNCEDTPEAGFFAVAVVKKSASDLTWDNLKGKKSCHTAVGRTAGWNIPMGLLYNKINHCRFDEFFSE
GCAPGSKKDSSLCKLCMGSGLNLCEPNNKEGYYGFTGAFRCLVEKGDVAFVKHQTVPQNTGGKNPDP
WAKNLNEKDYELLCLDGTRKPVEEYANCHLARAPNHAVVTRKDKEACVHKILRQQQHLFGSNVTDCSG
NFCLFRSETKDLLFRDDTVCLAKLHDRNTYEKYLGEEYVKAVGNLRKCSTSSLLEACTFRRP

SEQ ID NO: 7 Y95F/Y188F/Y517 N- and C-lobe mutant protein
VPDKTVRWCAVSEHEATKCQSFRDHMKSVIPSDGPSVACVKKASYLDCIRAIAANEADAVTLDAGLVYD
AYLAPNNLKPVVAEFYGSKEDPQTFFYAVAVVKKDSGFQMNQLRGKKSCHTGLGRSAGWNIPIGLLYCD
LPEPRKPLEKAVANFFSGSCAPCADGTDFPQLCQLCPGCGCSTLNQYFGFSGAFKCLKDGAGDVAFVK
HSTIFENLANKADRDQYELLCLDNTRKPVDEYKDCHLAQVPSHTVVARSMGGKEDLIWELLNQAQEHFG
KDKSKEFQLFSSPHGKDLLFKDSAHGFLKVPPRMDAKMYLGYEYVTAIRNLREGTCPEAPTDECKPVKW
CALSHHERLKCDEWSVNSVGKIECVSAETTEDCIAKIMNGEADAMSLDGGFVYIAGKCGLVPVLAENYNK
SDNCEDTPEAGYFAVAVVKKSASDLTWDNLKGKKSCHTAVGRTAGWNIPMGLLYNKINHCRFDEFFSE
GCAPGSKKDSSLCKLCMGSGLNLCEPNNKEGYYGFTGAFRCLVEKGDVAFVKHQTVPQNTGGKNPDP
WAKNLNEKDYELLCLDGTRKPVEEYANCHLARAPNHAVVTRKDKEACVHKILRQQQHLFGSNVTDCSG
NFCLFRSETKDLLFRDDTVCLAKLHDRNTYEKYLGEEYVKAVGNLRKCSTSSLLEACTFRRP

SEQ ID NO: 8 Y95F/Y188F/ Y426F/Y517F Transferrin N- and C-lobe mutant protein
VPDKTVRWCAVSEHEATKCQSFRDHMKSVIPSDGPSVACVKKASYLDCIRAIAANEADAVTLDAGLVYD
AYLAPNNLKPVVAEFYGSKEDPQTFFYAVAVVKKDSGFQMNQLRGKKSCHTGLGRSAGWNIPIGLLYCD
LPEPRKPLEKAVANFFSGSCAPCADGTDFPQLCQLCPGCGCSTLNQYFGFSGAFKCLKDGAGDVAFVK
HSTIFENLANKADRDQYELLCLDNTRKPVDEYKDCHLAQVPSHTVVARSMGGKEDLIWELLNQAQEHFG
KDKSKEFQLFSSPHGKDLLFKDSAHGFLKVPPRMDAKMYLGYEYVTAIRNLREGTCPEAPTDECKPVKW
CALSHHERLKCDEWSVNSVGKIECVSAETTEDCIAKIMNGEADAMSLDGGFVYIAGKCGLVPVLAENYNK
SDNCEDTPEAGFFAVAVVKKSASDLTWDNLKGKKSCHTAVGRTAGWNIPMGLLYNKINHCRFDEFFSE
GCAPGSKKDSSLCKLCMGSGLNLCEPNNKEGYYGFTGAFRCLVEKGDVAFVKHQTVPQNTGGKNPDP
WAKNLNEKDYELLCLDGTRKPVEEYANCHLARAPNHAVVTRKDKEACVHKILRQQQHLFGSNVTDCSG
NFCLFRSETKDLLFRDDTVCLAKLHDRNTYEKYLGEEYVKAVGNLRKCSTSSLLEACTFRRP

SEQ ID NO: 9 Y95F/Y188F/ Y426F/Y517F Transferrin-Fc N- and C-lobe mutant protein
VPDKTVRWCAVSEHEATKCQSFRDHMKSVIPSDGPSVACVKKASYLDCIRAIAANEADAVTLDAGLVYD
AYLAPNNLKPVVAEFYGSKEDPQTFFYAVAVVKKDSGFQMNQLRGKKSCHTGLGRSAGWNIPIGLLYCD
LPEPRKPLEKAVANFFSGSCAPCADGTDFPQLCQLCPGCGCSTLNQYFGFSGAFKCLKDGAGDVAFVK
HSTIFENLANKADRDQYELLCLDNTRKPVDEYKDCHLAQVPSHTVVARSMGGKEDLIWELLNQAQEHFG
KDKSKEFQLFSSPHGKDLLFKDSAHGFLKVPPRMDAKMYLGYEYVTAIRNLREGTCPEAPTDECKPVKW
CALSHHERLKCDEWSVNSVGKIECVSAETTEDCIAKIMNGEADAMSLDGGFVYIAGKCGLVPVLAENYNK
SDNCEDTPEAGFFAVAVVKKSASDLTWDNLKGKKSCHTAVGRTAGWNIPMGLLYNKINHCRFDEFFSE
GCAPGSKKDSSLCKLCMGSGLNLCEPNNKEGYYGFTGAFRCLVEKGDVAFVKHQTVPQNTGGKNPDP
WAKNLNEKDYELLCLDGTRKPVEEYANCHLARAPNHAVVTRKDKEACVHKILRQQQHLFGSNVTDCSG
NFCLFRSETKDLLFRDDTVCLAKLHDRNTYEKYLGEEYVKAVGNLRKCSTSSLLEACTFRRPDKTHTCPP
CPAPELLGGPSVFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSRDELTKNQVSLTC
LVKGFYPSDIAVEWESNGQPENNYKTTPPVLDSDGSFFLYSKLTVDKSRWQQGNVFSCSVMHEALHNH
YTQKSLSLSPGK

## Claims

1. A pharmaceutical composition comprising alpha-1 antitrypsin for the treatment of a neural cell injury arising from at least one of a traumatic brain injury, a non-traumatic brain injury, a spinal cord injury, or a peripheral nerve injury,
wherein the alpha-1 antitrypsin is administered at a dose of about 80 mg/kg/day to about 240 mg/kg/day as part of a multiple-dosage regimen.

2. The composition of claim 1 wherein the neural cell injury is caused by a traumatic brain injury or spinal cord injury arising from at least one of road a traffic accident, an assault, a sporting collision, or an unprotected fall.

3. The composition of claim 1 wherein the neural cell injury is caused by a non-traumatic brain injury is arising from at least one of a transient ischemic attack, an ischaemic stroke, a haemorrhagic stroke, cerebral hypoxia, cerebral anoxia, neonatal hypoxia, consumption of chemical toxins, hydrocephalus, meningitis, or encephalitis.

4. The composition of claim 1 wherein the neural cell injury is caused by an ischemic event selected from the group consisting of an transient ischemic attack, an ischemic stroke, cerebral hypoxia, cerebral anoxia, neonatal hypoxia, and combinations thereof.

5. The composition of claim 1 wherein the neural cell injury is caused by a stroke selected from the group consisting of a ischemic stroke and a haemorrhagic stroke.

6. The composition of claim 1 wherein the neural cell injury is caused by a spinal cord injury.

7. The composition of any preceding claim wherein the alpha-1 antitrypsin is administered as part of a combination therapy with at least one other plasma protein selected from the group consisting of:
albumin, antithrombin, transferrin, lactoferrin, polyclonal immunoglobulins, polyspecific immunoglobulins, C1 esterase inhibitor, transthyretin, and combinations thereof.

8. The composition of claim 7 whether the alpha-1 antitrypsin is administered as part of a combination therapy with transferrin.

9. The composition of claim 8 wherein the transferrin has an iron saturation of about 30% or less.

10. The composition of claim 7 wherein the alpha-1 antitrypsin is administered as part of a combination therapy with antithrombin.

11. The composition of claims 7 to 10 wherein the alpha-1 antitrypsin and the plasma protein are administered as a unitary dosage form.

12. The composition of any preceding claim wherein the alpha-1 antitrypsin is administered at a dose of about 80 mg/kg/day to about 240 mg/kg/day as part of a multiple-dosage regimen.

13. The composition of claim 12 wherein the alpha-1 antitrypsin is administered at a dose of about 80 mg/kg/day to about 120 mg/kg/day as part of a multiple-dosage regimen.

14. The composition of any preceding claim wherein the alpha-1 antitrypsin is administered at an initial dose of about 80 mg/kg to about 240 mg/kg on day 1 of a multiple-dosage regimen administration period, followed by a dose of about 80 mg/kg/day to about 240 mg/kg/day per dose during the remainder of the multiple dosing period.

15. The composition of any preceding claim wherein the alpha-1 antitrypsin is administered at an initial dose of about 80 mg/kg to about 120 mg/kg on day 1 of a multiple-dosage regimen administration period, followed by a dose of about 80 mg/kg/day to about 120 mg/kg/day per dose during the remainder of the multiple dosing period.

16. The composition of any preceding claim wherein the multiple dosing regimen comprises from about 2 to about 30 administrations up to a total cumulative dose.

17. The composition of any preceding claim wherein the multiple dosing regimen comprises from about 5 to about 20 administrations up to a total cumulative dose.

18. The composition of any preceding claim wherein the multiple dosing regimen extends over a period of about 1 to about 30 weeks.

19. The composition of any preceding claim wherein the multiple dosing regimen extends over a period of about 1 to about 10 weeks.

20. The composition of any preceding claim wherein the multiple dosing regimen comprises multiple portion doses administered at equally spaced intervals of from about 1 day to about 30 days.

21. The composition of any preceding claim wherein the multiple dosing regimen comprises multiple portion doses administered at equally spaced intervals of from about 1 day to about 10 days.

22. The composition of claims 1 to 19 wherein the multiple dosing regimen comprises multiple portion doses administered at unequally spaced intervals of from about 1 day to about 30 days.

23. The composition of claim 22 wherein the multiple dosing regimen comprises multiple portion doses administered at unequally spaced intervals of from about 1 day to about 10 days.

24. The composition of any preceding claim wherein the alpha-1 antitrypsin is administered by an administration route selected from the group consisting of intravenous, subcutaneous, intramuscular, intradermal, intraperitoneal, intrapulmonary, intranasal, and combinations thereof.

25. A pharmaceutical composition comprising alpha-1 antitrypsin for promoting spinal revascularisation in patient suffering from a spinal cord injury,
wherein the alpha-1 antitrypsin is administered at a dose of about 80 mg/kg/day to about 240 mg/kg/day as part of a multiple-dosage regimen.

26. The composition of claim 25 wherein the alpha-1 antitrypsin is administered at a dose of about 80 mg/kg/day to about 120 mg/kg/day as part of a multiple-dosage regimen.

27. The composition of claim 26 wherein the alpha-1 antitrypsin is administered at an initial dose of about 80 mg/kg to about 240 mg/kg on day 1 of a multiple-dosage regimen administration period, followed by a dose of about 80 mg/kg/day to about 240 mg/kg/day per dose during the remainder of the multiple dosing period.

28. The composition of claims 25 to 27 wherein the alpha-1 antitrypsin is administered at an initial dose of about 80 mg/kg to about 120 mg/kg on day 1 of a multiple-dosage regimen administration period, followed by a dose of about 80 mg/kg/day to about 120 mg/kg/day per dose during the remainder of the multiple dosing period.

29. The composition of claims 25 to 28 wherein the multiple dosing regimen comprises from about 2 to about 30 administrations up to a total cumulative dose.

30. The composition of claims 25 to 29 wherein the multiple dosing regimen comprises from about 5 to about 20 administrations up to a total cumulative dose.

31. The composition of claims 25 to 30 wherein the multiple dosing regimen extends over a period of about 1 to about 30 weeks.

32. The composition of claims 25 to 31 wherein the multiple dosing regimen extends over a period of about 1 to about 10 weeks.

33. The composition of claims 25 to 32 wherein the multiple dosing regimen comprises multiple portion doses administered at equally spaced intervals of from about 1 day to about 30 days.

34. The composition of claims 25 to 33 wherein the multiple dosing regimen comprises multiple portion doses administered at equally spaced intervals of from about 1 day to about 10 days.

35. The composition of claims 25 to 32 wherein the multiple dosing regimen comprises multiple portion doses administered at unequally spaced intervals of from about 1 day to about 30 days.

36. The composition of claims 25 to 32 wherein the multiple dosing regimen comprises multiple portion doses administered at unequally spaced intervals of from about 1 day to about 10 days.

37. The composition of claims 25 to 36 wherein the alpha-1 antitrypsin is administered by an administration route selected from the group consisting of intravenous, subcutaneous, intramuscular, intradermal, intraperitoneal, intrapulmonary, intranasal, and combinations thereof.

38. A pharmaceutical composition comprising alpha-1 antitrypsin for treating neuropathic pain in patient suffering from a spinal cord injury,
wherein the alpha-1 antitrypsin is administered at a dose of about 80 mg/kg/day to about 240 mg/kg/day as part of a multiple-dosage regimen.

39. The composition of claim 38 wherein the neuropathic pain is central neuropathic pain.

40. The composition of claim 38 wherein the neuropathic pain is peripheral neuropathic pain.

41. The composition of claims 38 to 40 wherein the alpha-1 antitrypsin is administered at a dose of about 80 mg/kg/day to about 120 mg/kg/day as part of a multiple-dosage regimen.

42. The composition of claims 38 to 40 wherein the alpha-1 antitrypsin is administered at an initial dose of about 80 mg/kg to about 240 mg/kg on day 1 of a multiple-dosage regimen administration period, followed by a dose of about 80 mg/kg/day to about 240 mg/kg/day per dose during the remainder of the multiple dosing period.

43. The composition of claims 38 to 40 wherein the alpha-1 antitrypsin is administered at an initial dose of about 80 mg/kg to about 120 mg/kg on day 1 of a multiple-dosage regimen administration period, followed by a dose of about 80 mg/kg/day to about 120 mg/kg/day per dose during the remainder of the multiple dosing period.

44. The composition of claims 38 to 43 wherein the multiple dosing regimen comprises from about 2 to about 30 administrations up to a total cumulative dose.

45. The composition of claims 38 to 44 wherein the multiple dosing regimen comprises from about 5 to about 20 administrations up to a total cumulative dose.

46. The composition of claims 38 to 45 wherein the multiple dosing regimen extends over a period of about 1 to about 30 weeks.

47. The composition of claims 38 to 46 wherein the multiple dosing regimen extends over a period of about 1 to about 10 weeks.

48. The composition of claims 38 to 47 wherein the multiple dosing regimen comprises multiple portion doses administered at equally spaced intervals of from about 1 day to about 30 days.

49. The composition of claims 38 to 48 wherein the multiple dosing regimen comprises multiple portion doses administered at equally spaced intervals of from about 1 day to about 10 days.

50. The composition of claims 38 to 47 wherein the multiple dosing regimen comprises multiple portion doses administered at unequally spaced intervals of from about 1 day to about 30 days.

51. The composition of claims 38 to 47 wherein the multiple dosing regimen comprises multiple portion doses administered at unequally spaced intervals of from about 1 day to about 10 days.

52. The composition of claims 38 to 51 wherein the alpha-1 antitrypsin is administered by an administration route selected from the group consisting of intravenous, subcutaneous, intramuscular, intradermal, intraperitoneal, intrapulmonary, intranasal, and combinations thereof.

53. Method of treatment of a neural cell injury arising from at least one of a traumatic brain injury, a non-traumatic brain injury, a spinal cord injury, or a peripheral nerve injury, comprising the administration of alpha-1 antitrypsin, wherein the alpha-1 antitrypsin is administered at a dose of about 80 mg/kg/day to about 240 mg/kg/day as part of a multiple-dosage regimen.

54. Method of treatment, according to claim 53, wherein the neural cell injury is caused by a traumatic brain injury or spinal cord injury arising from at least one of road a traffic accident, an assault, a sporting collision, or an unprotected fall.

55. Method of treatment, according to claim 53, wherein the neural cell injury is caused by a non-traumatic brain injury is arising from at least one of a transient ischemic attack, an ischaemic stroke, a haemorrhagic stroke, cerebral hypoxia, cerebral anoxia, neonatal hypoxia, consumption of chemical toxins, hydrocephalus, meningitis, or encephalitis.

56. Method of treatment, according to claim 53, wherein the neural cell injury is caused by an ischemic event selected from the group consisting of an transient ischemic attack, an ischemic stroke, cerebral hypoxia, cerebral anoxia, neonatal hypoxia, and combinations thereof.

57. Method of treatment, according to claim 53, wherein the neural cell injury is caused by a stroke selected from the group consisting of a ischemic stroke and a haemorrhagic stroke.

58. Method of treatment, according to claim 53, wherein the neural cell injury is caused by a spinal cord injury.

59. Method of treatment, according to any of claims 53-58, wherein the alpha-1 antitrypsin is administered as part of a combination therapy with at least one other plasma protein selected from the group consisting of:
albumin, antithrombin, transferrin, lactoferrin, polyclonal immunoglobulins, polyspecific immunoglobulins, C1 esterase inhibitor, transthyretin, and combinations thereof.

60. Method of treatment, according to claim 59, whether the alpha-1 antitrypsin is administered as part of a combination therapy with transferrin.

61. Method of treatment, according to claim 8, wherein the transferrin has an iron saturation of about 30% or less.

62. Method of treatment, according to claim 59, wherein the alpha-1 antitrypsin is administered as part of a combination therapy with antithrombin.

63. Method of treatment, according to any of claims 59-62, wherein the alpha-1 antitrypsin and the plasma protein are administered as a unitary dosage form.

64. Method of treatment, according to any of claims 53-63, wherein the alpha-1 antitrypsin is administered at a dose of about 80 mg/kg/day to about 240 mg/kg/day as part of a multiple-dosage regimen.

65. Method of treatment, according to claim 64, wherein the alpha-1 antitrypsin is administered at a dose of about 80 mg/kg/day to about 120 mg/kg/day as part of a multiple-dosage regimen.

66. Method of treatment, according to any of claims 53-65, wherein the alpha-1 antitrypsin is administered at an initial dose of about 80 mg/kg to about 240 mg/kg on day 1 of a multiple-dosage regimen administration period, followed by a dose of about 80 mg/kg/day to about 240 mg/kg/day per dose during the remainder of the multiple dosing period.

67. Method of treatment, according to any of claims 53-66, wherein the alpha-1 antitrypsin is administered at an initial dose of about 80 mg/kg to about 120 mg/kg on day 1 of a multiple-dosage regimen administration period, followed by a dose of about 80 mg/kg/day to about 120 mg/kg/day per dose during the remainder of the multiple dosing period.

68. Method of treatment, according to any of claims 53-67, wherein the multiple dosing regime comprises from about 2 to about 30 administrations up to a total cumulative dose.

69. Method of treatment, according to any of claims 53-68, wherein the multiple dosing regimen comprises from about 5 to about 20 administrations up to a total cumulative dose.

70. Method of treatment, according to any of claims 53-69, wherein the multiple dosing regimen extends over a period of about 1 to about 30 weeks.

71. Method of treatment, according to any of claims 53-70, wherein the multiple dosing regimen extends over a period of about 1 to about 10 weeks.

72. Method of treatment, according to any of claims 53-71, wherein the multiple dosing regimen comprises multiple portion doses administered at equally spaced intervals of from about 1 day to about 30 days.

73. Method of treatment, according to any of claims 53-72, wherein the multiple dosing regimen comprises multiple portion doses administered at equally spaced intervals of from about 1 day to about 10 days.

74. Method of treatment, according to any of claims 53-71, wherein the multiple dosing regimen comprises multiple portion doses administered at unequally spaced intervals of from about 1 day to about 30 days.

75. Method of treatment, according to claim 74, wherein the multiple dosing regimen comprises multiple portion doses administered at unequally spaced intervals of from about 1 day to about 10 days.

76. Method of treatment, according to any of claims 53-75, wherein the alpha-1 antitrypsin is administered by an administration route selected from the group consisting of intravenous, subcutaneous, intramuscular, intradermal, intraperitoneal, intrapulmonary, intranasal, and combinations thereof.

77. Method of treatment for promoting spinal revascularisation in patient suffering from a spinal cord injury, comprising administration of a pharmaceutical composition comprising alpha-1 antitrypsin, wherein the alpha-1 antitrypsin is administered at a dose of about 80 mg/kg/day to about 240 mg/kg/day as part of a multiple-dosage regimen.

78. Method of treatment, according to claim 77, wherein the alpha-1 antitrypsin is administered at a dose of about 80 mg/kg/day to about 120 mg/kg/day as part of a multiple-dosage regimen.

79. Method of treatment, according to claim 78, wherein the alpha-1 antitrypsin is administered at an initial dose of about 80 mg/kg to about 240 mg/kg on day 1 of a multiple-dosage regimen administration period, followed by a dose of about 80 mg/kg/day to about 240 mg/kg/day per dose during the remainder of the multiple dosing period.

80. Method of treatment, according to any of claims 77-79, wherein the alpha-1 antitrypsin is administered at an initial dose of about 80 mg/kg to about 120 mg/kg on day 1 of a multiple-dosage regimen administration period, followed by a dose of about 80 mg/kg/day to about 120 mg/kg/day per dose during the remainder of the multiple dosing period.

81. Method of treatment, according to any of claims 77-80, wherein the multiple dosing regimen comprises from about 2 to about 30 administrations up to a total cumulative dose.

82. Method of treatment, according to any of claims 77-81, wherein the multiple dosing regimen comprises from about 5 to about 20 administrations up to a total cumulative dose.

83. Method of treatment, according to any of claims 77-82, wherein the multiple dosing regimen extends over a period of about 1 to about 30 weeks.

84. Method of treatment, according to any of claims 77-83, wherein the multiple dosing regimen extends over a period of about 1 to about 10 weeks.

85. Method of treatment, according to any of claims 77-84, wherein the multiple dosing regimen comprises multiple portion doses administered at equally spaced intervals of from about 1 day to about 30 days.

86. Method of treatment, according to any of claims 77-85, wherein the multiple dosing regimen comprises multiple portion doses administered at equally spaced intervals of from about 1 day to about 10 days.

87. Method of treatment, according to any of claims 77-84, wherein the multiple dosing regimen comprises multiple portion doses administered at unequally spaced intervals of from about 1 day to about 30 days.

88. Method of treatment, according to any of claims 77-84, wherein the multiple dosing regimen comprises multiple portion doses administered at unequally spaced intervals of from about 1 day to about 10 days.

89. Method of treatment, according to any of claims 77-88, wherein the alpha-1 antitrypsin is administered by an administration route selected from the group consisting of intravenous, subcutaneous, intramuscular, intradermal, intraperitoneal, intrapulmonary, intranasal, and combinations thereof.

90. Method for treating neuropathic pain in patient suffering from a spinal cord injury, comprising administration of a composition comprising alpha-1 antitrypsin, wherein the alpha-1 antitrypsin is administered at a dose of about 80 mg/kg/day to about 240 mg/kg/day as part of a multiple-dosage regimen.

91. Method of treatment, according claim 90, wherein the neuropathic pain is central neuropathic pain.

92. Method of treatment, according claim 90, wherein the neuropathic pain is peripheral neuropathic pain.

93. Method of treatment, according to any of claims 90-92, wherein the alpha-1 antitrypsin is administered at a dose of about 80 mg/kg/day to about 120 mg/kg/day as part of a multiple-dosage regimen.

94. Method of treatment, according to any of claims 90-92, wherein the alpha-1 antitrypsin is administered at an initial dose of about 80 mg/kg to about 240 mg/kg on day 1 of a multiple-dosage regimen administration period, followed by a dose of about 80 mg/kg/day to about 240 mg/kg/day per dose during the remainder of the multiple dosing period.

95. Method of treatment, according to any of claims 90-92, wherein the alpha-1 antitrypsin is administered at an initial dose of about 80 mg/kg to about 120 mg/kg on day 1 of a multiple-dosage regimen administration period, followed by a dose of about 80 mg/kg/day to about 120 mg/kg/day per dose during the remainder of the multiple dosing period.

96. Method of treatment, according to any of claims 90-95, wherein the multiple dosing regimen comprises from about 2 to about 30 administrations up to a total cumulative dose.

97. Method of treatment, according to any of claims 90-96, wherein the multiple dosing regimen comprises from about 5 to about 20 administrations up to a total cumulative dose.

98. Method of treatment, according to any of claims 90-97, wherein the multiple dosing regimen extends over a period of about 1 to about 30 weeks.

99. Method of treatment, according to any of claims 90-98, wherein the multiple dosing regimen extends over a period of about 1 to about 10 weeks.

100. Method of treatment, according to any of claims 90-99, wherein the multiple dosing regimen comprises multiple portion doses administered at equally spaced intervals of from about 1 day to about 30 days.

101. Method of treatment, according to any of claims 90-100, wherein the multiple dosing regimen comprises multiple portion doses administered at equally spaced intervals of from about 1 day to about 10 days.

102. Method of treatment, according to any of claims 90-99, wherein the multiple dosing regimen comprises multiple portion doses administered at unequally spaced intervals of from about 1 day to about 30 days.

103. Method of treatment, according to any of claims 90-99, wherein the multiple dosing regimen comprises multiple portion doses administered at unequally spaced intervals of from about 1 day to about 10 days.

104. Method of treatment, according to any of claims 90-103, wherein wherein the alpha-1 antitrypsin is administered by an administration route selected from the group consisting of intravenous, subcutaneous, intramuscular, intradermal, intraperitoneal, intrapulmonary, intranasal, and combinations thereof.


SEQUENCE LISTING

<110>  GRIFOLS WORLDWIDE OPERATIONS LIMITED

<120>  Alpha-1 Antitrypsin Dosing Regimen

<130>  X-23333

<160>  9

<170>  PatentIn version 3.5

<210>  1
<211>  418
<212>  PRT
<213>  Homo sapiens

<400>  1

<210>  2
<211>  679
<212>  PRT
<213>  Homo sapiens

<400>  2

Val Pro Asp Lys Thr Val Arg Trp Cys Ala Val Ser Glu His Glu Ala
1               5                   10                  15

Thr Lys Cys Gln Ser Phe Arg Asp His Met Lys Ser Val Ile Pro Ser
            20                  25                  30

Asp Gly Pro Ser Val Ala Cys Val Lys Lys Ala Ser Tyr Leu Asp Cys
        35                  40                  45

Ile Arg Ala Ile Ala Ala Asn Glu Ala Asp Ala Val Thr Leu Asp Ala
    50                  55                  60

Gly Leu Val Tyr Asp Ala Tyr Leu Ala Pro Asn Asn Leu Lys Pro Val
65                  70                  75                  80

Val Ala Glu Phe Tyr Gly Ser Lys Glu Asp Pro Gln Thr Phe Tyr Tyr
                85                  90                  95

Ala Val Ala Val Val Lys Lys Asp Ser Gly Phe Gln Met Asn Gln Leu
            100                 105                 110

Arg Gly Lys Lys Ser Cys His Thr Gly Leu Gly Arg Ser Ala Gly Trp
        115                 120                 125

Asn Ile Pro Ile Gly Leu Leu Tyr Cys Asp Leu Pro Glu Pro Arg Lys
    130                 135                 140

Pro Leu Glu Lys Ala Val Ala Asn Phe Phe Ser Gly Ser Cys Ala Pro
145                 150                 155                 160

Cys Ala Asp Gly Thr Asp Phe Pro Gln Leu Cys Gln Leu Cys Pro Gly
                165                 170                 175

Cys Gly Cys Ser Thr Leu Asn Gln Tyr Phe Gly Tyr Ser Gly Ala Phe
            180                 185                 190

Lys Cys Leu Lys Asp Gly Ala Gly Asp Val Ala Phe Val Lys His Ser
        195                 200                 205

Thr Ile Phe Glu Asn Leu Ala Asn Lys Ala Asp Arg Asp Gln Tyr Glu
    210                 215                 220

Leu Leu Cys Leu Asp Asn Thr Arg Lys Pro Val Asp Glu Tyr Lys Asp
225                 230                 235                 240

Cys His Leu Ala Gln Val Pro Ser His Thr Val Val Ala Arg Ser Met
                245                 250                 255

Gly Gly Lys Glu Asp Leu Ile Trp Glu Leu Leu Asn Gln Ala Gln Glu
            260                 265                 270

His Phe Gly Lys Asp Lys Ser Lys Glu Phe Gln Leu Phe Ser Ser Pro
        275                 280                 285

His Gly Lys Asp Leu Leu Phe Lys Asp Ser Ala His Gly Phe Leu Lys
    290                 295                 300

Val Pro Pro Arg Met Asp Ala Lys Met Tyr Leu Gly Tyr Glu Tyr Val
305                 310                 315                 320

Thr Ala Ile Arg Asn Leu Arg Glu Gly Thr Cys Pro Glu Ala Pro Thr
                325                 330                 335

Asp Glu Cys Lys Pro Val Lys Trp Cys Ala Leu Ser His His Glu Arg
            340                 345                 350

Leu Lys Cys Asp Glu Trp Ser Val Asn Ser Val Gly Lys Ile Glu Cys
        355                 360                 365

Val Ser Ala Glu Thr Thr Glu Asp Cys Ile Ala Lys Ile Met Asn Gly
    370                 375                 380

Glu Ala Asp Ala Met Ser Leu Asp Gly Gly Phe Val Tyr Ile Ala Gly
385                 390                 395                 400

Lys Cys Gly Leu Val Pro Val Leu Ala Glu Asn Tyr Asn Lys Ser Asp
                405                 410                 415

Asn Cys Glu Asp Thr Pro Glu Ala Gly Tyr Phe Ala Val Ala Val Val
            420                 425                 430

Lys Lys Ser Ala Ser Asp Leu Thr Trp Asp Asn Leu Lys Gly Lys Lys
        435                 440                 445

Ser Cys His Thr Ala Val Gly Arg Thr Ala Gly Trp Asn Ile Pro Met
    450                 455                 460

Gly Leu Leu Tyr Asn Lys Ile Asn His Cys Arg Phe Asp Glu Phe Phe
465                 470                 475                 480

Ser Glu Gly Cys Ala Pro Gly Ser Lys Lys Asp Ser Ser Leu Cys Lys
                485                 490                 495

Leu Cys Met Gly Ser Gly Leu Asn Leu Cys Glu Pro Asn Asn Lys Glu
            500                 505                 510

Gly Tyr Tyr Gly Tyr Thr Gly Ala Phe Arg Cys Leu Val Glu Lys Gly
        515                 520                 525

Asp Val Ala Phe Val Lys His Gln Thr Val Pro Gln Asn Thr Gly Gly
    530                 535                 540

Lys Asn Pro Asp Pro Trp Ala Lys Asn Leu Asn Glu Lys Asp Tyr Glu
545                 550                 555                 560

Leu Leu Cys Leu Asp Gly Thr Arg Lys Pro Val Glu Glu Tyr Ala Asn
                565                 570                 575

Cys His Leu Ala Arg Ala Pro Asn His Ala Val Val Thr Arg Lys Asp
            580                 585                 590

Lys Glu Ala Cys Val His Lys Ile Leu Arg Gln Gln Gln His Leu Phe
        595                 600                 605

Gly Ser Asn Val Thr Asp Cys Ser Gly Asn Phe Cys Leu Phe Arg Ser
    610                 615                 620

Glu Thr Lys Asp Leu Leu Phe Arg Asp Asp Thr Val Cys Leu Ala Lys
625                 630                 635                 640

Leu His Asp Arg Asn Thr Tyr Glu Lys Tyr Leu Gly Glu Glu Tyr Val
                645                 650                 655

Lys Ala Val Gly Asn Leu Arg Lys Cys Ser Thr Ser Ser Leu Leu Glu
            660                 665                 670

Ala Cys Thr Phe Arg Arg Pro
        675

<210>  3
<211>  692
<212>  PRT
<213>  Homo sapiens

<400>  3

Gly Arg Arg Arg Arg Ser Val Gln Trp Cys Thr Val Ser Gln Pro Glu
1               5                   10                  15

Ala Thr Lys Cys Phe Gln Trp Gln Arg Asn Met Arg Arg Val Arg Gly
            20                  25                  30

Pro Pro Val Ser Cys Ile Lys Arg Asp Ser Pro Ile Gln Cys Ile Gln
        35                  40                  45

Ala Ile Ala Glu Asn Arg Ala Asp Ala Val Thr Leu Asp Gly Gly Phe
    50                  55                  60

Ile Tyr Glu Ala Gly Leu Ala Pro Tyr Lys Leu Arg Pro Val Ala Ala
65                  70                  75                  80

Glu Val Tyr Gly Thr Glu Arg Gln Pro Arg Thr His Tyr Tyr Ala Val
                85                  90                  95

Ala Val Val Lys Lys Gly Gly Ser Phe Gln Leu Asn Glu Leu Gln Gly
            100                 105                 110

Leu Lys Ser Cys His Thr Gly Leu Arg Arg Thr Ala Gly Trp Asn Val
        115                 120                 125

Pro Ile Gly Thr Leu Arg Pro Phe Leu Asn Trp Thr Gly Pro Pro Glu
    130                 135                 140

Pro Ile Glu Ala Ala Val Ala Arg Phe Phe Ser Ala Ser Cys Val Pro
145                 150                 155                 160

Gly Ala Asp Lys Gly Gln Phe Pro Asn Leu Cys Arg Leu Cys Ala Gly
                165                 170                 175

Thr Gly Glu Asn Lys Cys Ala Phe Ser Ser Gln Glu Pro Tyr Phe Ser
            180                 185                 190

Tyr Ser Gly Ala Phe Lys Cys Leu Arg Asp Gly Ala Gly Asp Val Ala
        195                 200                 205

Phe Ile Arg Glu Ser Thr Val Phe Glu Asp Leu Ser Asp Glu Ala Glu
    210                 215                 220

Arg Asp Glu Tyr Glu Leu Leu Cys Pro Asp Asn Thr Arg Lys Pro Val
225                 230                 235                 240

Asp Lys Phe Lys Asp Cys His Leu Ala Arg Val Pro Ser His Ala Val
                245                 250                 255

Val Ala Arg Ser Val Asn Gly Lys Glu Asp Ala Ile Trp Asn Leu Leu

260                 265                 270

Arg Gln Ala Gln Glu Lys Phe Gly Lys Asp Lys Ser Pro Lys Phe Gln
        275                 280                 285

Leu Phe Gly Ser Pro Ser Gly Gln Lys Asp Leu Leu Phe Lys Asp Ser
    290                 295                 300

Ala Ile Gly Phe Ser Arg Val Pro Pro Arg Ile Asp Ser Gly Leu Tyr
305                 310                 315                 320

Leu Gly Ser Gly Tyr Phe Thr Ala Ile Gln Asn Leu Arg Lys Ser Glu
                325                 330                 335

Glu Glu Val Ala Ala Arg Arg Ala Arg Val Val Trp Cys Ala Val Gly
            340                 345                 350

Glu Gln Glu Leu Arg Lys Cys Asn Gln Trp Ser Gly Leu Ser Glu Gly
        355                 360                 365

Ser Val Thr Cys Ser Ser Ala Ser Thr Thr Glu Asp Cys Ile Ala Leu
    370                 375                 380

Val Leu Lys Gly Glu Ala Asp Ala Met Ser Leu Asp Gly Gly Tyr Val
385                 390                 395                 400

Tyr Thr Ala Gly Lys Cys Gly Leu Val Pro Val Leu Ala Glu Asn Tyr
                405                 410                 415

Lys Ser Gln Gln Ser Ser Asp Pro Asp Pro Asn Cys Val Asp Arg Pro
            420                 425                 430

Val Glu Gly Tyr Leu Ala Val Ala Val Val Arg Arg Ser Asp Thr Ser
        435                 440                 445

Leu Thr Trp Asn Ser Val Lys Gly Lys Lys Ser Cys His Thr Ala Val
    450                 455                 460

Asp Arg Thr Ala Gly Trp Asn Ile Pro Met Gly Leu Leu Phe Asn Gln
465                 470                 475                 480

Thr Gly Ser Cys Lys Phe Asp Glu Tyr Phe Ser Gln Ser Cys Ala Pro
                485                 490                 495

Gly Ser Asp Pro Arg Ser Asn Leu Cys Ala Leu Cys Ile Gly Asp Glu
            500                 505                 510

Gln Gly Glu Asn Lys Cys Val Pro Asn Ser Asn Glu Arg Tyr Tyr Gly
        515                 520                 525

Tyr Thr Gly Ala Phe Arg Cys Leu Ala Glu Asn Ala Gly Asp Val Ala
    530                 535                 540

Phe Val Lys Asp Val Thr Val Leu Gln Asn Thr Asp Gly Asn Asn Asn
545                 550                 555                 560

Asp Ala Trp Ala Lys Asp Leu Lys Leu Ala Asp Phe Ala Leu Leu Cys
                565                 570                 575

Leu Asp Gly Lys Arg Lys Pro Val Thr Glu Ala Arg Ser Cys His Leu
            580                 585                 590

Ala Met Ala Pro Asn His Ala Val Val Ser Arg Met Asp Lys Val Glu
        595                 600                 605

Arg Leu Lys Gln Val Leu Leu His Gln Gln Ala Lys Phe Gly Arg Asn
    610                 615                 620

Gly Ser Asp Cys Pro Asp Lys Phe Cys Leu Phe Gln Ser Glu Thr Lys
625                 630                 635                 640

Asn Leu Leu Phe Asn Asp Asn Thr Glu Cys Leu Ala Arg Leu His Gly
                645                 650                 655

Lys Thr Thr Tyr Glu Lys Tyr Leu Gly Pro Gln Tyr Val Ala Gly Ile
            660                 665                 670

Thr Asn Leu Lys Lys Cys Ser Thr Ser Pro Leu Leu Glu Ala Cys Glu
        675                 680                 685

Phe Leu Arg Lys
    690

<210>  4
<211>  679
<212>  PRT
<213>  Artificial Sequence

<220>
<223>  Mutant protein

<400>  4

Val Pro Asp Lys Thr Val Arg Trp Cys Ala Val Ser Glu His Glu Ala
1               5                   10                  15

Thr Lys Cys Gln Ser Phe Arg Asp His Met Lys Ser Val Ile Pro Ser

20                  25                  30

Asp Gly Pro Ser Val Ala Cys Val Lys Lys Ala Ser Tyr Leu Asp Cys
        35                  40                  45

Ile Arg Ala Ile Ala Ala Asn Glu Ala Asp Ala Val Thr Leu Asp Ala
    50                  55                  60

Gly Leu Val Tyr Asp Ala Tyr Leu Ala Pro Asn Asn Leu Lys Pro Val
65                  70                  75                  80

Val Ala Glu Phe Tyr Gly Ser Lys Glu Asp Pro Gln Thr Phe Tyr Tyr
                85                  90                  95

Ala Val Ala Val Val Lys Lys Asp Ser Gly Phe Gln Met Asn Gln Leu
            100                 105                 110

Arg Gly Lys Lys Ser Cys His Thr Gly Leu Gly Arg Ser Ala Gly Trp
        115                 120                 125

Asn Ile Pro Ile Gly Leu Leu Tyr Cys Asp Leu Pro Glu Pro Arg Lys
    130                 135                 140

Pro Leu Glu Lys Ala Val Ala Asn Phe Phe Ser Gly Ser Cys Ala Pro
145                 150                 155                 160

Cys Ala Asp Gly Thr Asp Phe Pro Gln Leu Cys Gln Leu Cys Pro Gly
                165                 170                 175

Cys Gly Cys Ser Thr Leu Asn Gln Tyr Phe Gly Phe Ser Gly Ala Phe
            180                 185                 190

Lys Cys Leu Lys Asp Gly Ala Gly Asp Val Ala Phe Val Lys His Ser
        195                 200                 205

Thr Ile Phe Glu Asn Leu Ala Asn Lys Ala Asp Arg Asp Gln Tyr Glu
    210                 215                 220

Leu Leu Cys Leu Asp Asn Thr Arg Lys Pro Val Asp Glu Tyr Lys Asp
225                 230                 235                 240

Cys His Leu Ala Gln Val Pro Ser His Thr Val Val Ala Arg Ser Met
                245                 250                 255

Gly Gly Lys Glu Asp Leu Ile Trp Glu Leu Leu Asn Gln Ala Gln Glu
            260                 265                 270

His Phe Gly Lys Asp Lys Ser Lys Glu Phe Gln Leu Phe Ser Ser Pro
        275                 280                 285

His Gly Lys Asp Leu Leu Phe Lys Asp Ser Ala His Gly Phe Leu Lys
    290                 295                 300

Val Pro Pro Arg Met Asp Ala Lys Met Tyr Leu Gly Tyr Glu Tyr Val
305                 310                 315                 320

Thr Ala Ile Arg Asn Leu Arg Glu Gly Thr Cys Pro Glu Ala Pro Thr
                325                 330                 335

Asp Glu Cys Lys Pro Val Lys Trp Cys Ala Leu Ser His His Glu Arg
            340                 345                 350

Leu Lys Cys Asp Glu Trp Ser Val Asn Ser Val Gly Lys Ile Glu Cys
        355                 360                 365

Val Ser Ala Glu Thr Thr Glu Asp Cys Ile Ala Lys Ile Met Asn Gly
    370                 375                 380

Glu Ala Asp Ala Met Ser Leu Asp Gly Gly Phe Val Tyr Ile Ala Gly
385                 390                 395                 400

Lys Cys Gly Leu Val Pro Val Leu Ala Glu Asn Tyr Asn Lys Ser Asp
                405                 410                 415

Asn Cys Glu Asp Thr Pro Glu Ala Gly Tyr Phe Ala Val Ala Val Val
            420                 425                 430

Lys Lys Ser Ala Ser Asp Leu Thr Trp Asp Asn Leu Lys Gly Lys Lys
        435                 440                 445

Ser Cys His Thr Ala Val Gly Arg Thr Ala Gly Trp Asn Ile Pro Met
    450                 455                 460

Gly Leu Leu Tyr Asn Lys Ile Asn His Cys Arg Phe Asp Glu Phe Phe
465                 470                 475                 480

Ser Glu Gly Cys Ala Pro Gly Ser Lys Lys Asp Ser Ser Leu Cys Lys
                485                 490                 495

Leu Cys Met Gly Ser Gly Leu Asn Leu Cys Glu Pro Asn Asn Lys Glu
            500                 505                 510

Gly Tyr Tyr Gly Tyr Thr Gly Ala Phe Arg Cys Leu Val Glu Lys Gly
        515                 520                 525

Asp Val Ala Phe Val Lys His Gln Thr Val Pro Gln Asn Thr Gly Gly
    530                 535                 540

Lys Asn Pro Asp Pro Trp Ala Lys Asn Leu Asn Glu Lys Asp Tyr Glu
545                 550                 555                 560

Leu Leu Cys Leu Asp Gly Thr Arg Lys Pro Val Glu Glu Tyr Ala Asn
                565                 570                 575

Cys His Leu Ala Arg Ala Pro Asn His Ala Val Val Thr Arg Lys Asp
            580                 585                 590

Lys Glu Ala Cys Val His Lys Ile Leu Arg Gln Gln Gln His Leu Phe
        595                 600                 605

Gly Ser Asn Val Thr Asp Cys Ser Gly Asn Phe Cys Leu Phe Arg Ser
    610                 615                 620

Glu Thr Lys Asp Leu Leu Phe Arg Asp Asp Thr Val Cys Leu Ala Lys
625                 630                 635                 640

Leu His Asp Arg Asn Thr Tyr Glu Lys Tyr Leu Gly Glu Glu Tyr Val
                645                 650                 655

Lys Ala Val Gly Asn Leu Arg Lys Cys Ser Thr Ser Ser Leu Leu Glu
            660                 665                 670

Ala Cys Thr Phe Arg Arg Pro
        675

<210>  5
<211>  679
<212>  PRT
<213>  Artificial Sequence

<220>
<223>  Mutant protein

<400>  5

Val Pro Asp Lys Thr Val Arg Trp Cys Ala Val Ser Glu His Glu Ala
1               5                   10                  15

Thr Lys Cys Gln Ser Phe Arg Asp His Met Lys Ser Val Ile Pro Ser
            20                  25                  30

Asp Gly Pro Ser Val Ala Cys Val Lys Lys Ala Ser Tyr Leu Asp Cys
        35                  40                  45

Ile Arg Ala Ile Ala Ala Asn Glu Ala Asp Ala Val Thr Leu Asp Ala
    50                  55                  60

Gly Leu Val Tyr Asp Ala Tyr Leu Ala Pro Asn Asn Leu Lys Pro Val
65                  70                  75                  80

Val Ala Glu Phe Tyr Gly Ser Lys Glu Asp Pro Gln Thr Phe Phe Tyr
                85                  90                  95

Ala Val Ala Val Val Lys Lys Asp Ser Gly Phe Gln Met Asn Gln Leu
            100                 105                 110

Arg Gly Lys Lys Ser Cys His Thr Gly Leu Gly Arg Ser Ala Gly Trp
        115                 120                 125

Asn Ile Pro Ile Gly Leu Leu Tyr Cys Asp Leu Pro Glu Pro Arg Lys
    130                 135                 140

Pro Leu Glu Lys Ala Val Ala Asn Phe Phe Ser Gly Ser Cys Ala Pro
145                 150                 155                 160

Cys Ala Asp Gly Thr Asp Phe Pro Gln Leu Cys Gln Leu Cys Pro Gly
                165                 170                 175

Cys Gly Cys Ser Thr Leu Asn Gln Tyr Phe Gly Phe Ser Gly Ala Phe
            180                 185                 190

Lys Cys Leu Lys Asp Gly Ala Gly Asp Val Ala Phe Val Lys His Ser
        195                 200                 205

Thr Ile Phe Glu Asn Leu Ala Asn Lys Ala Asp Arg Asp Gln Tyr Glu
    210                 215                 220

Leu Leu Cys Leu Asp Asn Thr Arg Lys Pro Val Asp Glu Tyr Lys Asp
225                 230                 235                 240

Cys His Leu Ala Gln Val Pro Ser His Thr Val Val Ala Arg Ser Met
                245                 250                 255

Gly Gly Lys Glu Asp Leu Ile Trp Glu Leu Leu Asn Gln Ala Gln Glu
            260                 265                 270

His Phe Gly Lys Asp Lys Ser Lys Glu Phe Gln Leu Phe Ser Ser Pro
        275                 280                 285

His Gly Lys Asp Leu Leu Phe Lys Asp Ser Ala His Gly Phe Leu Lys
    290                 295                 300

Val Pro Pro Arg Met Asp Ala Lys Met Tyr Leu Gly Tyr Glu Tyr Val
305                 310                 315                 320

Thr Ala Ile Arg Asn Leu Arg Glu Gly Thr Cys Pro Glu Ala Pro Thr
                325                 330                 335

Asp Glu Cys Lys Pro Val Lys Trp Cys Ala Leu Ser His His Glu Arg
            340                 345                 350

Leu Lys Cys Asp Glu Trp Ser Val Asn Ser Val Gly Lys Ile Glu Cys
        355                 360                 365

Val Ser Ala Glu Thr Thr Glu Asp Cys Ile Ala Lys Ile Met Asn Gly
    370                 375                 380

Glu Ala Asp Ala Met Ser Leu Asp Gly Gly Phe Val Tyr Ile Ala Gly
385                 390                 395                 400

Lys Cys Gly Leu Val Pro Val Leu Ala Glu Asn Tyr Asn Lys Ser Asp
                405                 410                 415

Asn Cys Glu Asp Thr Pro Glu Ala Gly Tyr Phe Ala Val Ala Val Val
            420                 425                 430

Lys Lys Ser Ala Ser Asp Leu Thr Trp Asp Asn Leu Lys Gly Lys Lys
        435                 440                 445

Ser Cys His Thr Ala Val Gly Arg Thr Ala Gly Trp Asn Ile Pro Met
    450                 455                 460

Gly Leu Leu Tyr Asn Lys Ile Asn His Cys Arg Phe Asp Glu Phe Phe
465                 470                 475                 480

Ser Glu Gly Cys Ala Pro Gly Ser Lys Lys Asp Ser Ser Leu Cys Lys
                485                 490                 495

Leu Cys Met Gly Ser Gly Leu Asn Leu Cys Glu Pro Asn Asn Lys Glu
            500                 505                 510

Gly Tyr Tyr Gly Tyr Thr Gly Ala Phe Arg Cys Leu Val Glu Lys Gly
        515                 520                 525

Asp Val Ala Phe Val Lys His Gln Thr Val Pro Gln Asn Thr Gly Gly
    530                 535                 540

Lys Asn Pro Asp Pro Trp Ala Lys Asn Leu Asn Glu Lys Asp Tyr Glu
545                 550                 555                 560

Leu Leu Cys Leu Asp Gly Thr Arg Lys Pro Val Glu Glu Tyr Ala Asn
                565                 570                 575

Cys His Leu Ala Arg Ala Pro Asn His Ala Val Val Thr Arg Lys Asp
            580                 585                 590

Lys Glu Ala Cys Val His Lys Ile Leu Arg Gln Gln Gln His Leu Phe
        595                 600                 605

Gly Ser Asn Val Thr Asp Cys Ser Gly Asn Phe Cys Leu Phe Arg Ser
    610                 615                 620

Glu Thr Lys Asp Leu Leu Phe Arg Asp Asp Thr Val Cys Leu Ala Lys
625                 630                 635                 640

Leu His Asp Arg Asn Thr Tyr Glu Lys Tyr Leu Gly Glu Glu Tyr Val
                645                 650                 655

Lys Ala Val Gly Asn Leu Arg Lys Cys Ser Thr Ser Ser Leu Leu Glu
            660                 665                 670

Ala Cys Thr Phe Arg Arg Pro
        675

<210>  6
<211>  679
<212>  PRT
<213>  Artificial Sequence

<220>
<223>  Mutant protein

<400>  6

Val Pro Asp Lys Thr Val Arg Trp Cys Ala Val Ser Glu His Glu Ala
1               5                   10                  15

Thr Lys Cys Gln Ser Phe Arg Asp His Met Lys Ser Val Ile Pro Ser
            20                  25                  30

Asp Gly Pro Ser Val Ala Cys Val Lys Lys Ala Ser Tyr Leu Asp Cys
        35                  40                  45

Ile Arg Ala Ile Ala Ala Asn Glu Ala Asp Ala Val Thr Leu Asp Ala
    50                  55                  60

Gly Leu Val Tyr Asp Ala Tyr Leu Ala Pro Asn Asn Leu Lys Pro Val
65                  70                  75                  80

Val Ala Glu Phe Tyr Gly Ser Lys Glu Asp Pro Gln Thr Phe Tyr Tyr
                85                  90                  95

Ala Val Ala Val Val Lys Lys Asp Ser Gly Phe Gln Met Asn Gln Leu
            100                 105                 110

Arg Gly Lys Lys Ser Cys His Thr Gly Leu Gly Arg Ser Ala Gly Trp
        115                 120                 125

Asn Ile Pro Ile Gly Leu Leu Tyr Cys Asp Leu Pro Glu Pro Arg Lys
    130                 135                 140

Pro Leu Glu Lys Ala Val Ala Asn Phe Phe Ser Gly Ser Cys Ala Pro
145                 150                 155                 160

Cys Ala Asp Gly Thr Asp Phe Pro Gln Leu Cys Gln Leu Cys Pro Gly
                165                 170                 175

Cys Gly Cys Ser Thr Leu Asn Gln Tyr Phe Gly Tyr Ser Gly Ala Phe
            180                 185                 190

Lys Cys Leu Lys Asp Gly Ala Gly Asp Val Ala Phe Val Lys His Ser
        195                 200                 205

Thr Ile Phe Glu Asn Leu Ala Asn Lys Ala Asp Arg Asp Gln Tyr Glu
    210                 215                 220

Leu Leu Cys Leu Asp Asn Thr Arg Lys Pro Val Asp Glu Tyr Lys Asp
225                 230                 235                 240

Cys His Leu Ala Gln Val Pro Ser His Thr Val Val Ala Arg Ser Met
                245                 250                 255

Gly Gly Lys Glu Asp Leu Ile Trp Glu Leu Leu Asn Gln Ala Gln Glu
            260                 265                 270

His Phe Gly Lys Asp Lys Ser Lys Glu Phe Gln Leu Phe Ser Ser Pro
        275                 280                 285

His Gly Lys Asp Leu Leu Phe Lys Asp Ser Ala His Gly Phe Leu Lys
    290                 295                 300

Val Pro Pro Arg Met Asp Ala Lys Met Tyr Leu Gly Tyr Glu Tyr Val
305                 310                 315                 320

Thr Ala Ile Arg Asn Leu Arg Glu Gly Thr Cys Pro Glu Ala Pro Thr
                325                 330                 335

Asp Glu Cys Lys Pro Val Lys Trp Cys Ala Leu Ser His His Glu Arg
            340                 345                 350

Leu Lys Cys Asp Glu Trp Ser Val Asn Ser Val Gly Lys Ile Glu Cys
        355                 360                 365

Val Ser Ala Glu Thr Thr Glu Asp Cys Ile Ala Lys Ile Met Asn Gly
    370                 375                 380

Glu Ala Asp Ala Met Ser Leu Asp Gly Gly Phe Val Tyr Ile Ala Gly
385                 390                 395                 400

Lys Cys Gly Leu Val Pro Val Leu Ala Glu Asn Tyr Asn Lys Ser Asp
                405                 410                 415

Asn Cys Glu Asp Thr Pro Glu Ala Gly Phe Phe Ala Val Ala Val Val
            420                 425                 430

Lys Lys Ser Ala Ser Asp Leu Thr Trp Asp Asn Leu Lys Gly Lys Lys
        435                 440                 445

Ser Cys His Thr Ala Val Gly Arg Thr Ala Gly Trp Asn Ile Pro Met
    450                 455                 460

Gly Leu Leu Tyr Asn Lys Ile Asn His Cys Arg Phe Asp Glu Phe Phe
465                 470                 475                 480

Ser Glu Gly Cys Ala Pro Gly Ser Lys Lys Asp Ser Ser Leu Cys Lys
                485                 490                 495

Leu Cys Met Gly Ser Gly Leu Asn Leu Cys Glu Pro Asn Asn Lys Glu
            500                 505                 510

Gly Tyr Tyr Gly Phe Thr Gly Ala Phe Arg Cys Leu Val Glu Lys Gly
        515                 520                 525

Asp Val Ala Phe Val Lys His Gln Thr Val Pro Gln Asn Thr Gly Gly
    530                 535                 540

Lys Asn Pro Asp Pro Trp Ala Lys Asn Leu Asn Glu Lys Asp Tyr Glu
545                 550                 555                 560

Leu Leu Cys Leu Asp Gly Thr Arg Lys Pro Val Glu Glu Tyr Ala Asn
                565                 570                 575

Cys His Leu Ala Arg Ala Pro Asn His Ala Val Val Thr Arg Lys Asp

580                 585                 590

Lys Glu Ala Cys Val His Lys Ile Leu Arg Gln Gln Gln His Leu Phe
        595                 600                 605

Gly Ser Asn Val Thr Asp Cys Ser Gly Asn Phe Cys Leu Phe Arg Ser
    610                 615                 620

Glu Thr Lys Asp Leu Leu Phe Arg Asp Asp Thr Val Cys Leu Ala Lys
625                 630                 635                 640

Leu His Asp Arg Asn Thr Tyr Glu Lys Tyr Leu Gly Glu Glu Tyr Val
                645                 650                 655

Lys Ala Val Gly Asn Leu Arg Lys Cys Ser Thr Ser Ser Leu Leu Glu
            660                 665                 670

Ala Cys Thr Phe Arg Arg Pro
        675

<210>  7
<211>  679
<212>  PRT
<213>  Artificial Sequence

<220>
<223>  Mutant protein

<400>  7

Val Pro Asp Lys Thr Val Arg Trp Cys Ala Val Ser Glu His Glu Ala
1               5                   10                  15

Thr Lys Cys Gln Ser Phe Arg Asp His Met Lys Ser Val Ile Pro Ser
            20                  25                  30

Asp Gly Pro Ser Val Ala Cys Val Lys Lys Ala Ser Tyr Leu Asp Cys
        35                  40                  45

Ile Arg Ala Ile Ala Ala Asn Glu Ala Asp Ala Val Thr Leu Asp Ala
    50                  55                  60

Gly Leu Val Tyr Asp Ala Tyr Leu Ala Pro Asn Asn Leu Lys Pro Val
65                  70                  75                  80

Val Ala Glu Phe Tyr Gly Ser Lys Glu Asp Pro Gln Thr Phe Phe Tyr
                85                  90                  95

Ala Val Ala Val Val Lys Lys Asp Ser Gly Phe Gln Met Asn Gln Leu
            100                 105                 110

Arg Gly Lys Lys Ser Cys His Thr Gly Leu Gly Arg Ser Ala Gly Trp
        115                 120                 125

Asn Ile Pro Ile Gly Leu Leu Tyr Cys Asp Leu Pro Glu Pro Arg Lys
    130                 135                 140

Pro Leu Glu Lys Ala Val Ala Asn Phe Phe Ser Gly Ser Cys Ala Pro
145                 150                 155                 160

Cys Ala Asp Gly Thr Asp Phe Pro Gln Leu Cys Gln Leu Cys Pro Gly
                165                 170                 175

Cys Gly Cys Ser Thr Leu Asn Gln Tyr Phe Gly Phe Ser Gly Ala Phe
            180                 185                 190

Lys Cys Leu Lys Asp Gly Ala Gly Asp Val Ala Phe Val Lys His Ser
        195                 200                 205

Thr Ile Phe Glu Asn Leu Ala Asn Lys Ala Asp Arg Asp Gln Tyr Glu
    210                 215                 220

Leu Leu Cys Leu Asp Asn Thr Arg Lys Pro Val Asp Glu Tyr Lys Asp
225                 230                 235                 240

Cys His Leu Ala Gln Val Pro Ser His Thr Val Val Ala Arg Ser Met
                245                 250                 255

Gly Gly Lys Glu Asp Leu Ile Trp Glu Leu Leu Asn Gln Ala Gln Glu
            260                 265                 270

His Phe Gly Lys Asp Lys Ser Lys Glu Phe Gln Leu Phe Ser Ser Pro
        275                 280                 285

His Gly Lys Asp Leu Leu Phe Lys Asp Ser Ala His Gly Phe Leu Lys
    290                 295                 300

Val Pro Pro Arg Met Asp Ala Lys Met Tyr Leu Gly Tyr Glu Tyr Val
305                 310                 315                 320

Thr Ala Ile Arg Asn Leu Arg Glu Gly Thr Cys Pro Glu Ala Pro Thr
                325                 330                 335

Asp Glu Cys Lys Pro Val Lys Trp Cys Ala Leu Ser His His Glu Arg
            340                 345                 350

Leu Lys Cys Asp Glu Trp Ser Val Asn Ser Val Gly Lys Ile Glu Cys

        355                 360                 365

Val Ser Ala Glu Thr Thr Glu Asp Cys Ile Ala Lys Ile Met Asn Gly
    370                 375                 380

Glu Ala Asp Ala Met Ser Leu Asp Gly Gly Phe Val Tyr Ile Ala Gly
385                 390                 395                 400

Lys Cys Gly Leu Val Pro Val Leu Ala Glu Asn Tyr Asn Lys Ser Asp
                405                 410                 415

Asn Cys Glu Asp Thr Pro Glu Ala Gly Tyr Phe Ala Val Ala Val Val
            420                 425                 430

Lys Lys Ser Ala Ser Asp Leu Thr Trp Asp Asn Leu Lys Gly Lys Lys
        435                 440                 445

Ser Cys His Thr Ala Val Gly Arg Thr Ala Gly Trp Asn Ile Pro Met
    450                 455                 460

Gly Leu Leu Tyr Asn Lys Ile Asn His Cys Arg Phe Asp Glu Phe Phe
465                 470                 475                 480

Ser Glu Gly Cys Ala Pro Gly Ser Lys Lys Asp Ser Ser Leu Cys Lys
                485                 490                 495

Leu Cys Met Gly Ser Gly Leu Asn Leu Cys Glu Pro Asn Asn Lys Glu
            500                 505                 510

Gly Tyr Tyr Gly Phe Thr Gly Ala Phe Arg Cys Leu Val Glu Lys Gly
        515                 520                 525

Asp Val Ala Phe Val Lys His Gln Thr Val Pro Gln Asn Thr Gly Gly
    530                 535                 540

Lys Asn Pro Asp Pro Trp Ala Lys Asn Leu Asn Glu Lys Asp Tyr Glu
545                 550                 555                 560

Leu Leu Cys Leu Asp Gly Thr Arg Lys Pro Val Glu Glu Tyr Ala Asn
                565                 570                 575

Cys His Leu Ala Arg Ala Pro Asn His Ala Val Val Thr Arg Lys Asp
            580                 585                 590

Lys Glu Ala Cys Val His Lys Ile Leu Arg Gln Gln Gln His Leu Phe
        595                 600                 605

Gly Ser Asn Val Thr Asp Cys Ser Gly Asn Phe Cys Leu Phe Arg Ser
    610                 615                 620

Glu Thr Lys Asp Leu Leu Phe Arg Asp Asp Thr Val Cys Leu Ala Lys
625                 630                 635                 640

Leu His Asp Arg Asn Thr Tyr Glu Lys Tyr Leu Gly Glu Glu Tyr Val
                645                 650                 655

Lys Ala Val Gly Asn Leu Arg Lys Cys Ser Thr Ser Ser Leu Leu Glu
            660                 665                 670

Ala Cys Thr Phe Arg Arg Pro
        675

<210>  8
<211>  679
<212>  PRT
<213>  Artificial Sequence

<220>
<223>  Mutant protein

<400>  8

Val Pro Asp Lys Thr Val Arg Trp Cys Ala Val Ser Glu His Glu Ala
1               5                   10                  15

Thr Lys Cys Gln Ser Phe Arg Asp His Met Lys Ser Val Ile Pro Ser
            20                  25                  30

Asp Gly Pro Ser Val Ala Cys Val Lys Lys Ala Ser Tyr Leu Asp Cys
        35                  40                  45

Ile Arg Ala Ile Ala Ala Asn Glu Ala Asp Ala Val Thr Leu Asp Ala
    50                  55                  60

Gly Leu Val Tyr Asp Ala Tyr Leu Ala Pro Asn Asn Leu Lys Pro Val
65                  70                  75                  80

Val Ala Glu Phe Tyr Gly Ser Lys Glu Asp Pro Gln Thr Phe Phe Tyr
                85                  90                  95

Ala Val Ala Val Val Lys Lys Asp Ser Gly Phe Gln Met Asn Gln Leu
            100                 105                 110

Arg Gly Lys Lys Ser Cys His Thr Gly Leu Gly Arg Ser Ala Gly Trp
        115                 120                 125

Asn Ile Pro Ile Gly Leu Leu Tyr Cys Asp Leu Pro Glu Pro Arg Lys

130                 135                 140

Pro Leu Glu Lys Ala Val Ala Asn Phe Phe Ser Gly Ser Cys Ala Pro
145                 150                 155                 160

Cys Ala Asp Gly Thr Asp Phe Pro Gln Leu Cys Gln Leu Cys Pro Gly
                165                 170                 175

Cys Gly Cys Ser Thr Leu Asn Gln Tyr Phe Gly Phe Ser Gly Ala Phe
            180                 185                 190

Lys Cys Leu Lys Asp Gly Ala Gly Asp Val Ala Phe Val Lys His Ser
        195                 200                 205

Thr Ile Phe Glu Asn Leu Ala Asn Lys Ala Asp Arg Asp Gln Tyr Glu
    210                 215                 220

Leu Leu Cys Leu Asp Asn Thr Arg Lys Pro Val Asp Glu Tyr Lys Asp
225                 230                 235                 240

Cys His Leu Ala Gln Val Pro Ser His Thr Val Val Ala Arg Ser Met
                245                 250                 255

Gly Gly Lys Glu Asp Leu Ile Trp Glu Leu Leu Asn Gln Ala Gln Glu
            260                 265                 270

His Phe Gly Lys Asp Lys Ser Lys Glu Phe Gln Leu Phe Ser Ser Pro
        275                 280                 285

His Gly Lys Asp Leu Leu Phe Lys Asp Ser Ala His Gly Phe Leu Lys
    290                 295                 300

Val Pro Pro Arg Met Asp Ala Lys Met Tyr Leu Gly Tyr Glu Tyr Val
305                 310                 315                 320

Thr Ala Ile Arg Asn Leu Arg Glu Gly Thr Cys Pro Glu Ala Pro Thr
                325                 330                 335

Asp Glu Cys Lys Pro Val Lys Trp Cys Ala Leu Ser His His Glu Arg
            340                 345                 350

Leu Lys Cys Asp Glu Trp Ser Val Asn Ser Val Gly Lys Ile Glu Cys
        355                 360                 365

Val Ser Ala Glu Thr Thr Glu Asp Cys Ile Ala Lys Ile Met Asn Gly
    370                 375                 380

Glu Ala Asp Ala Met Ser Leu Asp Gly Gly Phe Val Tyr Ile Ala Gly
385                 390                 395                 400

Lys Cys Gly Leu Val Pro Val Leu Ala Glu Asn Tyr Asn Lys Ser Asp
                405                 410                 415

Asn Cys Glu Asp Thr Pro Glu Ala Gly Phe Phe Ala Val Ala Val Val
            420                 425                 430

Lys Lys Ser Ala Ser Asp Leu Thr Trp Asp Asn Leu Lys Gly Lys Lys
        435                 440                 445

Ser Cys His Thr Ala Val Gly Arg Thr Ala Gly Trp Asn Ile Pro Met
    450                 455                 460

Gly Leu Leu Tyr Asn Lys Ile Asn His Cys Arg Phe Asp Glu Phe Phe
465                 470                 475                 480

Ser Glu Gly Cys Ala Pro Gly Ser Lys Lys Asp Ser Ser Leu Cys Lys
                485                 490                 495

Leu Cys Met Gly Ser Gly Leu Asn Leu Cys Glu Pro Asn Asn Lys Glu
            500                 505                 510

Gly Tyr Tyr Gly Phe Thr Gly Ala Phe Arg Cys Leu Val Glu Lys Gly
        515                 520                 525

Asp Val Ala Phe Val Lys His Gln Thr Val Pro Gln Asn Thr Gly Gly
    530                 535                 540

Lys Asn Pro Asp Pro Trp Ala Lys Asn Leu Asn Glu Lys Asp Tyr Glu
545                 550                 555                 560

Leu Leu Cys Leu Asp Gly Thr Arg Lys Pro Val Glu Glu Tyr Ala Asn
                565                 570                 575

Cys His Leu Ala Arg Ala Pro Asn His Ala Val Val Thr Arg Lys Asp
            580                 585                 590

Lys Glu Ala Cys Val His Lys Ile Leu Arg Gln Gln Gln His Leu Phe
        595                 600                 605

Gly Ser Asn Val Thr Asp Cys Ser Gly Asn Phe Cys Leu Phe Arg Ser
    610                 615                 620

Glu Thr Lys Asp Leu Leu Phe Arg Asp Asp Thr Val Cys Leu Ala Lys
625                 630                 635                 640

Leu His Asp Arg Asn Thr Tyr Glu Lys Tyr Leu Gly Glu Glu Tyr Val
                645                 650                 655

Lys Ala Val Gly Asn Leu Arg Lys Cys Ser Thr Ser Ser Leu Leu Glu
            660                 665                 670

Ala Cys Thr Phe Arg Arg Pro
        675

<210>  9
<211>  906
<212>  PRT
<213>  Artificial Sequence

<220>
<223>  Mutant protein

<400>  9

Val Pro Asp Lys Thr Val Arg Trp Cys Ala Val Ser Glu His Glu Ala
1               5                   10                  15

Thr Lys Cys Gln Ser Phe Arg Asp His Met Lys Ser Val Ile Pro Ser
            20                  25                  30

Asp Gly Pro Ser Val Ala Cys Val Lys Lys Ala Ser Tyr Leu Asp Cys
        35                  40                  45

Ile Arg Ala Ile Ala Ala Asn Glu Ala Asp Ala Val Thr Leu Asp Ala
    50                  55                  60

Gly Leu Val Tyr Asp Ala Tyr Leu Ala Pro Asn Asn Leu Lys Pro Val
65                  70                  75                  80

Val Ala Glu Phe Tyr Gly Ser Lys Glu Asp Pro Gln Thr Phe Phe Tyr
                85                  90                  95

Ala Val Ala Val Val Lys Lys Asp Ser Gly Phe Gln Met Asn Gln Leu
            100                 105                 110

Arg Gly Lys Lys Ser Cys His Thr Gly Leu Gly Arg Ser Ala Gly Trp
        115                 120                 125

Asn Ile Pro Ile Gly Leu Leu Tyr Cys Asp Leu Pro Glu Pro Arg Lys
    130                 135                 140

Pro Leu Glu Lys Ala Val Ala Asn Phe Phe Ser Gly Ser Cys Ala Pro
145                 150                 155                 160

Cys Ala Asp Gly Thr Asp Phe Pro Gln Leu Cys Gln Leu Cys Pro Gly
                    165                 170                 175

Cys Gly Cys Ser Thr Leu Asn Gln Tyr Phe Gly Phe Ser Gly Ala Phe
                180                 185                 190

Lys Cys Leu Lys Asp Gly Ala Gly Asp Val Ala Phe Val Lys His Ser
            195                 200                 205

Thr Ile Phe Glu Asn Leu Ala Asn Lys Ala Asp Arg Asp Gln Tyr Glu
    210                 215                 220

Leu Leu Cys Leu Asp Asn Thr Arg Lys Pro Val Asp Glu Tyr Lys Asp
225                 230                 235                 240

Cys His Leu Ala Gln Val Pro Ser His Thr Val Val Ala Arg Ser Met
                245                 250                 255

Gly Gly Lys Glu Asp Leu Ile Trp Glu Leu Leu Asn Gln Ala Gln Glu
                260                 265                 270

His Phe Gly Lys Asp Lys Ser Lys Glu Phe Gln Leu Phe Ser Ser Pro
            275                 280                 285

His Gly Lys Asp Leu Leu Phe Lys Asp Ser Ala His Gly Phe Leu Lys
    290                 295                 300

Val Pro Pro Arg Met Asp Ala Lys Met Tyr Leu Gly Tyr Glu Tyr Val
305                 310                 315                 320

Thr Ala Ile Arg Asn Leu Arg Glu Gly Thr Cys Pro Glu Ala Pro Thr
                325                 330                 335

Asp Glu Cys Lys Pro Val Lys Trp Cys Ala Leu Ser His His Glu Arg
                340                 345                 350

Leu Lys Cys Asp Glu Trp Ser Val Asn Ser Val Gly Lys Ile Glu Cys
            355                 360                 365

Val Ser Ala Glu Thr Thr Glu Asp Cys Ile Ala Lys Ile Met Asn Gly
    370                 375                 380

Glu Ala Asp Ala Met Ser Leu Asp Gly Gly Phe Val Tyr Ile Ala Gly
385                 390                 395                 400

Lys Cys Gly Leu Val Pro Val Leu Ala Glu Asn Tyr Asn Lys Ser Asp
                405                 410                 415

Asn Cys Glu Asp Thr Pro Glu Ala Gly Phe Phe Ala Val Ala Val Val
        420                 425                 430

Lys Lys Ser Ala Ser Asp Leu Thr Trp Asp Asn Leu Lys Gly Lys Lys
        435                 440                 445

Ser Cys His Thr Ala Val Gly Arg Thr Ala Gly Trp Asn Ile Pro Met
    450                 455                 460

Gly Leu Leu Tyr Asn Lys Ile Asn His Cys Arg Phe Asp Glu Phe Phe
465                 470                 475                 480

Ser Glu Gly Cys Ala Pro Gly Ser Lys Lys Asp Ser Ser Leu Cys Lys
                485                 490                 495

Leu Cys Met Gly Ser Gly Leu Asn Leu Cys Glu Pro Asn Asn Lys Glu
            500                 505                 510

Gly Tyr Tyr Gly Phe Thr Gly Ala Phe Arg Cys Leu Val Glu Lys Gly
        515                 520                 525

Asp Val Ala Phe Val Lys His Gln Thr Val Pro Gln Asn Thr Gly Gly
    530                 535                 540

Lys Asn Pro Asp Pro Trp Ala Lys Asn Leu Asn Glu Lys Asp Tyr Glu
545                 550                 555                 560

Leu Leu Cys Leu Asp Gly Thr Arg Lys Pro Val Glu Glu Tyr Ala Asn
                565                 570                 575

Cys His Leu Ala Arg Ala Pro Asn His Ala Val Val Thr Arg Lys Asp
            580                 585                 590

Lys Glu Ala Cys Val His Lys Ile Leu Arg Gln Gln Gln His Leu Phe
        595                 600                 605

Gly Ser Asn Val Thr Asp Cys Ser Gly Asn Phe Cys Leu Phe Arg Ser
    610                 615                 620

Glu Thr Lys Asp Leu Leu Phe Arg Asp Asp Thr Val Cys Leu Ala Lys
625                 630                 635                 640

Leu His Asp Arg Asn Thr Tyr Glu Lys Tyr Leu Gly Glu Glu Tyr Val
                645                 650                 655

Lys Ala Val Gly Asn Leu Arg Lys Cys Ser Thr Ser Ser Leu Leu Glu
            660                 665                 670

Ala Cys Thr Phe Arg Arg Pro Asp Lys Thr His Thr Cys Pro Pro Cys
        675                 680                 685

Pro Ala Pro Glu Leu Leu Gly Gly Pro Ser Val Phe Leu Phe Pro Pro
    690                 695                 700

Lys Pro Lys Asp Thr Leu Met Ile Ser Arg Thr Pro Glu Val Thr Cys
705                 710                 715                 720

Val Val Val Asp Val Ser His Glu Asp Pro Glu Val Lys Phe Asn Trp
                725                 730                 735

Tyr Val Asp Gly Val Glu Val His Asn Ala Lys Thr Lys Pro Arg Glu
            740                 745                 750

Glu Gln Tyr Asn Ser Thr Tyr Arg Val Val Ser Val Leu Thr Val Leu
        755                 760                 765

His Gln Asp Trp Leu Asn Gly Lys Glu Tyr Lys Cys Lys Val Ser Asn
    770                 775                 780

Lys Ala Leu Pro Ala Pro Ile Glu Lys Thr Ile Ser Lys Ala Lys Gly
785                 790                 795                 800

Gln Pro Arg Glu Pro Gln Val Tyr Thr Leu Pro Pro Ser Arg Asp Glu
                805                 810                 815

Leu Thr Lys Asn Gln Val Ser Leu Thr Cys Leu Val Lys Gly Phe Tyr
            820                 825                 830

Pro Ser Asp Ile Ala Val Glu Trp Glu Ser Asn Gly Gln Pro Glu Asn
        835                 840                 845

Asn Tyr Lys Thr Thr Pro Pro Val Leu Asp Ser Asp Gly Ser Phe Phe
    850                 855                 860

Leu Tyr Ser Lys Leu Thr Val Asp Lys Ser Arg Trp Gln Gln Gly Asn
865                 870                 875                 880

Val Phe Ser Cys Ser Val Met His Glu Ala Leu His Asn His Tyr Thr
                885                 890                 895

Gln Lys Ser Leu Ser Leu Ser Pro Gly Lys
            900                 905